# EUROPEAN PATENT APPLICATION

(11) **EP 4 647 066 A1**
(43) Date of publication of application: **12.11.2025**
(21) Application number: 25175132.7
(22) Date of filing: 08.05.2025
(51) Int. Cl.: A61K 9/00, A61K 9/08, A61K 47/06, A61K 47/34, A61K 47/44

(54) **A COMPOSITION COMPRISING A MIXTURE OF AT LEAST ONE OIL AND ONE OR MORE SEMIFLUORINATED ALKANES, A USE OF THE COMPOSITION FOR TREATING A DISEASE AS WELL AS A METHOD OF PRODUCING THE COMPOSITION AND CORRESPONDING KITS**

(30) Priority: 08.05.2024 EP 24174853
(71) Applicant: Fluoron GmbH, 89077 Ulm (DE)
(72) Inventor: MANGER, Markus, 89231 Neu-Ulm (DE); HAMMER, Maximilian, 69115 Heidelberg (DE); AUFFARTH, Gerd Uwe, 69198 Schriesheim (DE); UHL, Philipp, 69120 Heidelberg (DE)
(74) Representative: Schiweck Weinzierl Koch Patentanwälte Partnerschaft mbB

(57) **Abstract**

The present invention inter alia relates to a composition comprising a mixture of at least one oil and one or more semifluorinated alkanes, the refractive index of the composition being from about 1.310 to about 1.365, and to a use of said composition for treating a disease, wherein the disease optionally comprises treating a detached retina. The invention also relates to a kit of parts comprising the composition, and to a method of producing the composition.

## Description

### FIELD OF THE INVENTION

The present invention relates to a composition comprising a mixture of at least one oil and one or more semifluorinated alkane(s). The invention also relates to the composition for use in the treatment of a disease, as well as to a method of producing the composition, and to a kit of parts comprising the composition.

### BACKGROUND OF THE INVENTION

Retinal detachment is the separation of the neurosensory retina from the underlying pigment epithelium. Untreated retinal detachment can lead to permanent vision loss or blindness. Retinal detachment is caused by traction of the vitreous upon the retina. The traction can be 'dynamic', caused by eye movements and thus relative movement of the vitreous and the retina; or 'static', due to contraction of membranes on the surface of the retina. Retinal detachments are associated with myopia, pseudophakia, trauma and diabetes; it is often the common pathway leading to blindness in a host of ophthalmic eye diseases.

When retinal detachment is accompanied by retinal breaks (also known as perforations, holes, or tears), fluid from the vitreous cavity enters the subretinal space. This form of retinal detachment is called "rhegmatogenous".

In general, retinal detachment can be treated, for example, by pneumatic retinopexy, in which a gas bubble is injected into the vitreous cavity to help push the retina back against the wall of the eye. This method can also be used in conjunction with laser and cryo-surgical techniques if necessary. The gases preferentially used for such operations are usually either hexafluoroethane (C₂F₆), octafluoropropane (C₃F₈) or sulfur hexafluoride (SF₆), which, when mixed with sterile air, may remain in the eye for long periods of time. Sooner or later, the gas used is typically replaced by the eye's own natural fluid, although recently there have been concerns about the toxicology of fluorine-based compositions.

Another commonly used treatment procedure is vitrectomy. This involves removing all or part of the vitreous gel from the eye and replacing it with a tamponing agent, such as a perfluorocarbon liquid (herein sometimes called "perfluorocarbons"), silicone oil, or a gas. When a gas is used, the eye fills with the body's own fluid over time. In this technique, a small incision is made in the sclera and the vitreous gel is removed by means of a small suction-cutting device. When the vitreous gel is removed, a saline solution is used to maintain pressure through a continuous infusion. An air/gas mixture is then injected.

Alternatively, perfluorocarbon liquids, semifluorinated alkanes or alkenes (e.g., in combination with silicone oil as described herein), and more commonly silicone oils are injected as tamponade agents. The tamponade agent is an immiscible liquid, especially with aqueous media/liquids, which prevents retinal breaks due to its interfacial tension and buoyancy. The tamponade material is therefore intended to close the retinal tear and re-establish the retina on the underlying pigment epithelium.

However, these mentioned tamponades will need to be removed later. While perfluorocarbons are most used as intraoperative tamponades due to cytotoxicity concerns, silicone oils are commonly used as long-term tamponades.

During the time of use of the tamponade, patients often experience a drop in visual acuity. Furthermore, functional limitations and complaints due to anisometropia, anisoeikonia and loss of binocularity have been reported. In some cases, patients who normally do not need any correction of visual acuity are - during the application of the tamponade - obliged to wear corrective lenses, such as glasses or contact lenses. Other patients who usually use e.g. glasses or contact lenses to correct visual acuity are often obliged to exchange the prescription of their glasses/lenses to a large extent during the treatment period. Even when corrective lenses are used, visual acuity is decreased due to the nature of the movement of the tamponade within the eye. These corrective measures are needed as all currently used long-term endotamponades do not have a suitable refractive error.

It is therefore an object of the present invention to address one or more problems associated with the prior art procedures and in particular to provide a composition/ a tamponade agent that can be effectively used in the treatment of eyes disease such as retinal detachment. Furthermore, it is an object of the present invention to provide a composition/ a tamponade agent that resists emulsification or substantially prevents emulsification from occurring and is well tolerated in the eye and being - at the same time - more user friendly with regard to the above described issues.

### SUMMARY OF THE INVENTION

This object is inter alia accomplished by the compositions, the uses, the methods and the kits having the features of the respective independent claims.

In a first aspect, the invention provides a composition comprising a mixture of at least one oil and one or more semifluorinated alkanes, the refractive index of the composition being from about 1.310 to about 1.365.

The refractive index as referred to herein relates to the refractive index of the composition at 35 °C and with respect to a wavelength of 589 nm, the wavelength of sodium D-line.

By the provision of a composition suitable to be used as a tamponade and having the refractive index as herein provided, the above-mentioned problems can be reduced/avoided. In particular, it has surprisingly been found that it is possible to create compositions suitable to be used as tamponade composition wherein the refractive index of the composition is closer to -optionally even identical with -a refractory index of the natural vitreous body of the eye than refractive indexes of prior art tamponade agents comprising silicone oils. Thus, the compositions as herein provided maintain the advantage of using oils like e.g. silicone oils in tamponade agents while at the same time reduce and or avoid the above-mentioned problems related to visual acuity during the use of the tamponade.

In a second aspect, the invention provides the composition for use in the treatment of a disease, such as e.g. the treatment of a detached retina.

In a third aspect, the invention provides a kit of parts comprising the composition as herein provided.

In a fourth aspect, the invention provides a method of producing a composition for use in the treatment of a detached retina, comprising mixing at least one oils and one or more semifluorinated alkanes to produce a composition as provided according to the first aspect.

In a fifth aspect, the invention provides a method for treating a disease, e.g. a retinal detachment, in a subject in need thereof, comprising administering the composition according to the first aspect into the vitreous cavity of the eye of said subject.

In a sixth aspect, the invention provides a use of the composition of the first aspect for treating of a disease, such as a detached retina, optionally wherein the use comprises the use of the composition as temporary tamponade.

In a seventh aspect the invention provides a method for treating retinal detachment in a subject in need thereof, the subject further suffering from ametropia, comprising administering a composition into the vitreous cavity of the eye of said subject; wherein the composition comprises a mixture of at least one oil in a first quantity and one or more semifluorinated alkanes in a second quantity, and further wherein the method comprises the step of selecting the first quantity of the at least one oil and the second quantity of the one or more semifluorinated alkanes, thereby adjusting a refractive index of the mixture such that the administered composition corrects the ametropia of the subject.

### BRIEF DESCRIPTION OF THE DRAWINGS

The invention will be better understood with reference to the detailed description when considered in conjunction with the non-limiting examples and the drawings, in which:

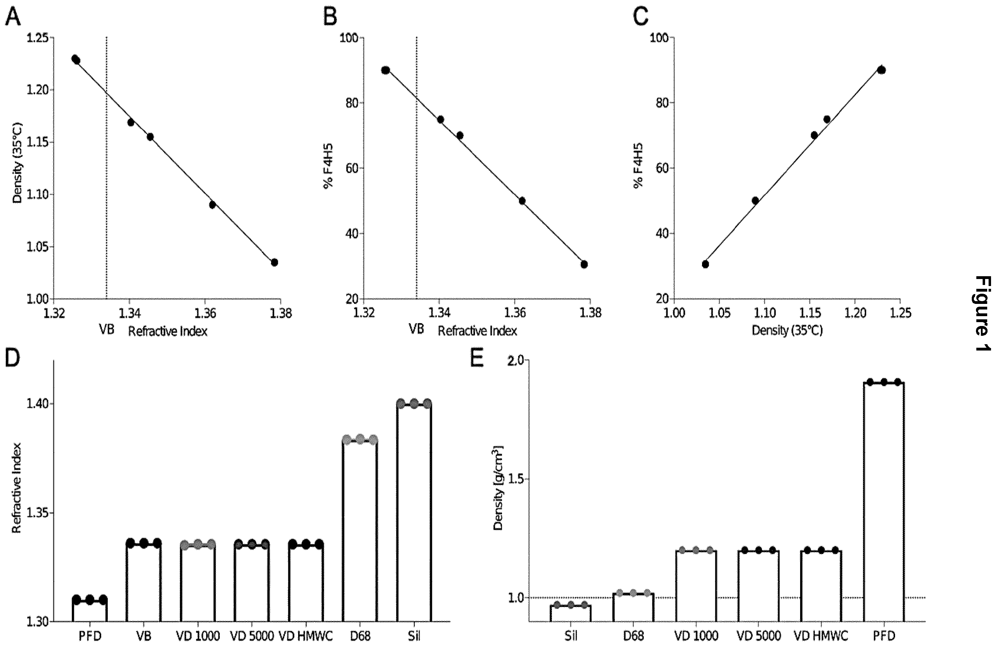

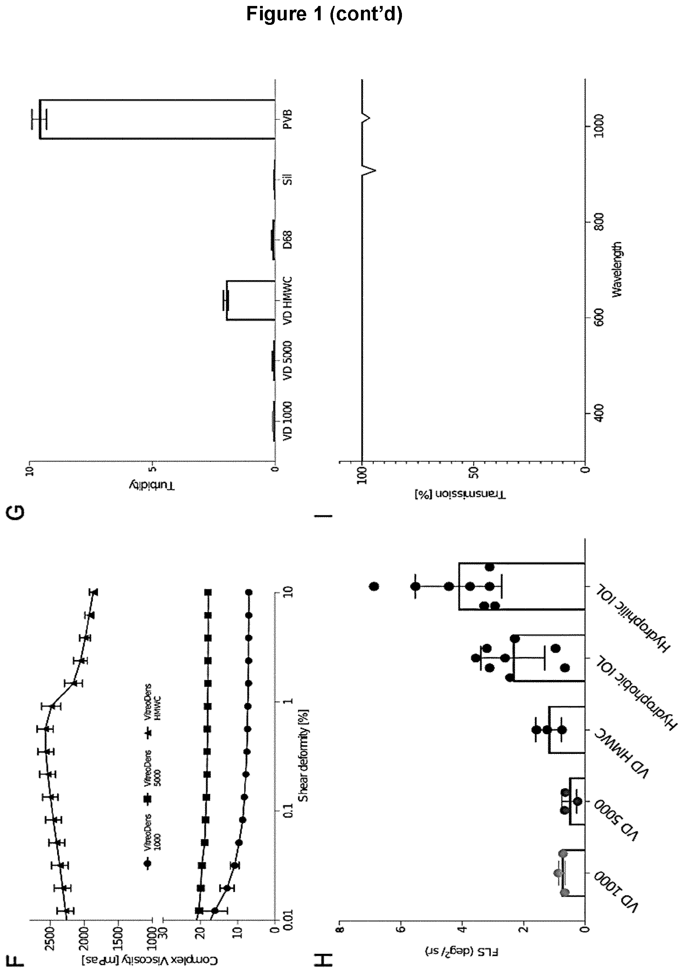

**Fig. 1** shows a graphical representation of the results of assays for determining physico-chemical properties of a composition according to an embodiment of the present invention comprising 80% w/w perfluorobutylpentane (F4H5), and 20% w/w polydimethylsiloxane (PDMS). **Fig. 1A** shows the relation between the density of a composition and the refractive index for compositions having different F4H5: PDMS ratios. **Fig. 1B** shows the relation between the percentage of F4H5 of a composition and the refractive index for compositions having different F4H5: PDMS ratios. **Fig. 1C** shows the relation between the percentage of F4H5 of a composition and the density for compositions having different F4H5: PDMS ratios. **Fig.** 1D shows the refractive index of compositions according to embodiments of the present invention in comparison to the refractive index of currently used endotamponades and heavy liquids according to the state of the art. **Fig. 1E** shows the density of compositions according to embodiments of the present invention in comparison to the density of currently used endotamponades and heavy liquids according to the state of the art. **Fig. 1F** shows the shear deformity in dependency of the complex viscosity for three compositions with varied molecular weight of the PDMS-component according to embodiments of the present invention. **Fig. 1G** shows the turbidity of compositions according to embodiments of the present invention in comparison to the turbidity of currently used endotamponades and heavy liquids according to the state of the art. **Fig. 1H** shows the forward light scattering (FLS) of compositions according to embodiments of the present invention in comparison to the forward light scattering (FLS) of other biomaterials according to the state of the art. **Fig. 1I** shows a transmission spectrum of a composition according to an embodiment of the present invention comprising 80% w/w perfluorobutylpentane (F4H5), and 20% w/w polydimethylsiloxane (PDMS).

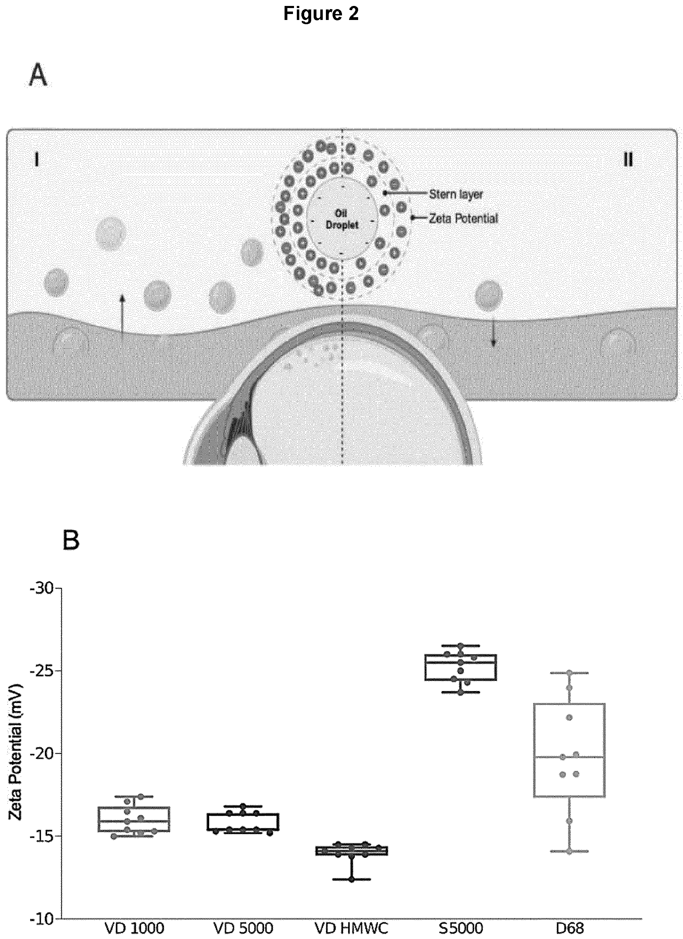

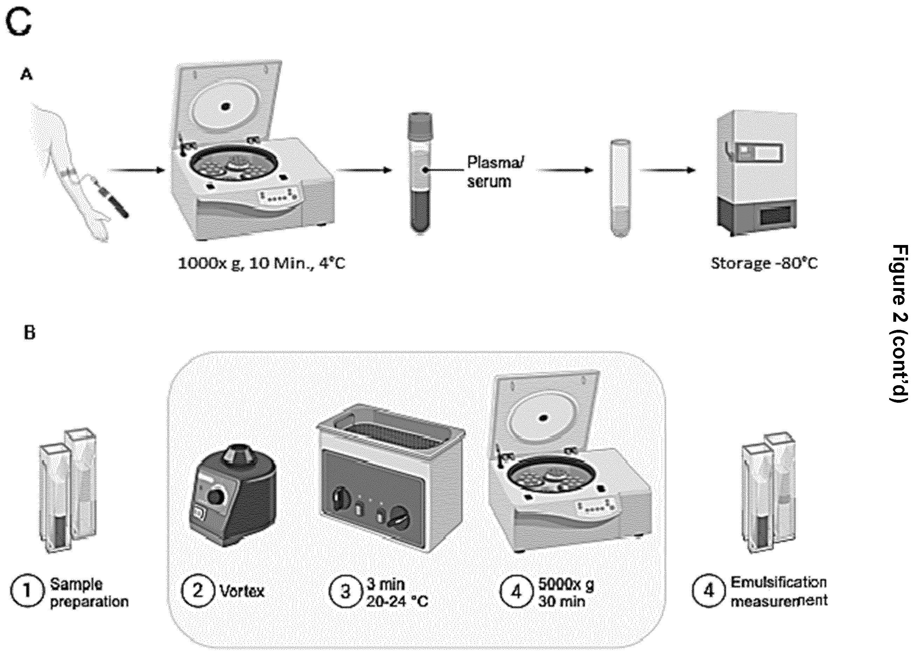

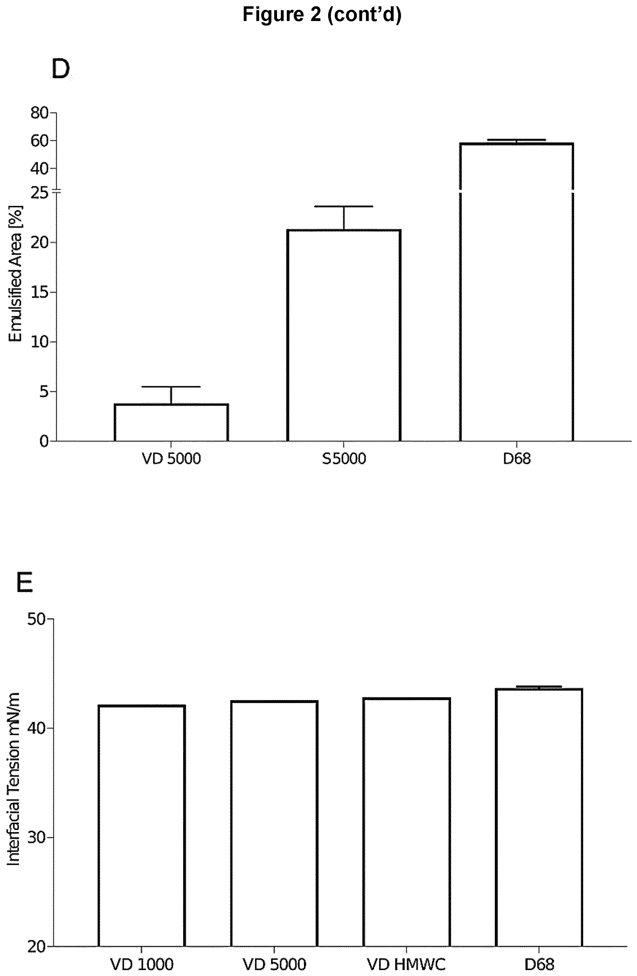

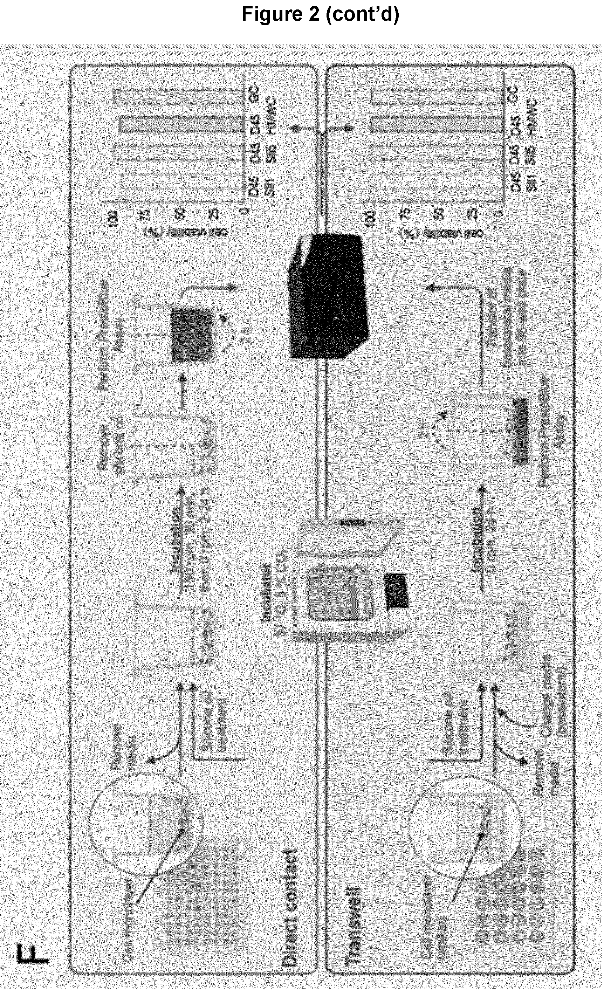

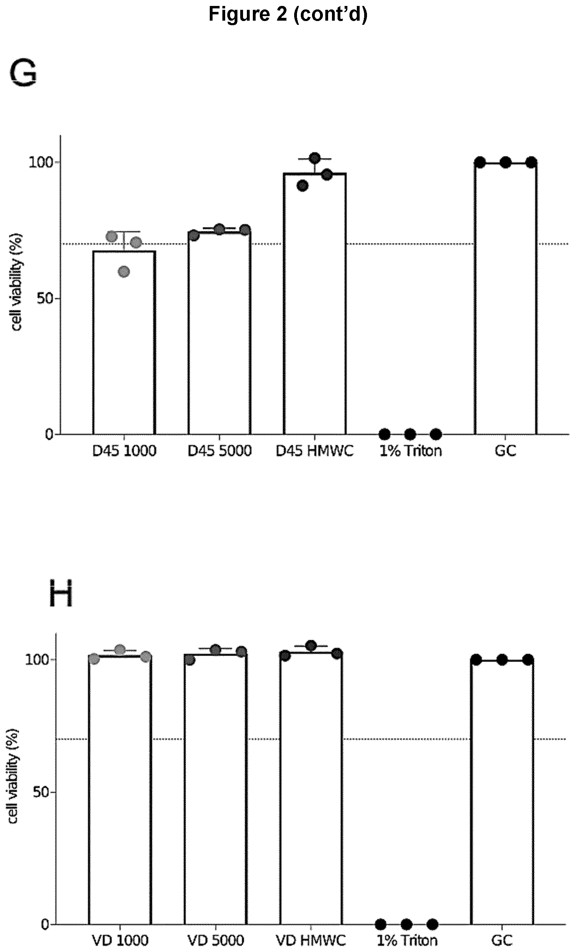

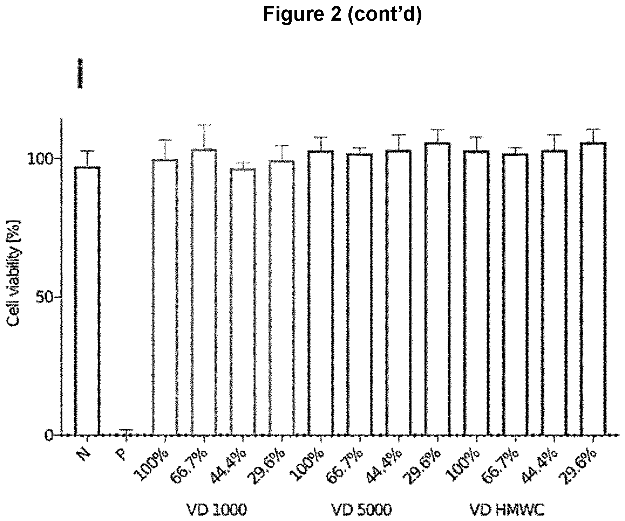

**Fig. 2** shows a graphical representation of further results of assays for determining physico-chemical properties of a composition according to an embodiment of the present invention comprising 80% w/w perfluorobutylpentane (F4H5), and 20% w/w polydimethylsiloxane (PDMS). **Fig. 2A** graphically illustrates the principle correlation between a composition's tendency to emulsify and its electrochemical emulsion stability using zeta-potential. **Fig. 2B** shows the emulsion stability of compositions according to an embodiment of the present invention comprising 80% w/w perfluorobutylpentane (F4H5), and 20% w/w polydimethylsiloxane (PDMS) in comparison to the emulsion stability of currently used endotamponades and heavy liquids according to the state of the art. emulsion stability. **Fig. 2C** graphically illustrates the principle of the Caramoy-Assay for measuring emulsification. **Fig. 2D** shows the emulsion stability of a composition according to an embodiment of the present invention comprising 80% w/w perfluorobutylpentane (F4H5), and 20% w/w polydimethylsiloxane (PDMS) in comparison to the emulsion stability of currently used endotamponades according to the state of the art. **Fig. 2E** shows the interfacial tension of compositions according to an embodiment of the present invention comprising 80% w/w perfluorobutylpentane (F4H5), and 20% w/w polydimethylsiloxane (PDMS) in comparison to the interfacial tensions of currently used endotamponades. **Fig. 2F** graphically illustrates the principle assay for testing biocompatibility and non-cytotoxicity of samples in cell cultures using both the direct and transwell approach. **Fig. 2G** shows the cell viability of compositions according to an embodiment of the present invention comprising 80% w/w perfluorobutylpentane (F4H5), and 20% w/w polydimethylsiloxane (PDMS). **Fig. 2** **H** shows the cell viability of compositions according to an embodiment of the present invention comprising 80% w/w perfluorobutylpentane (F4H5), and 20% w/w polydimethylsiloxane (PDMS). **Fig. 2I** shows the cell viability of compositions according to an embodiment of the present invention comprising 80% w/w perfluorobutylpentane (F4H5), and 20% w/w polydimethylsiloxane (PDMS), using different percentages.

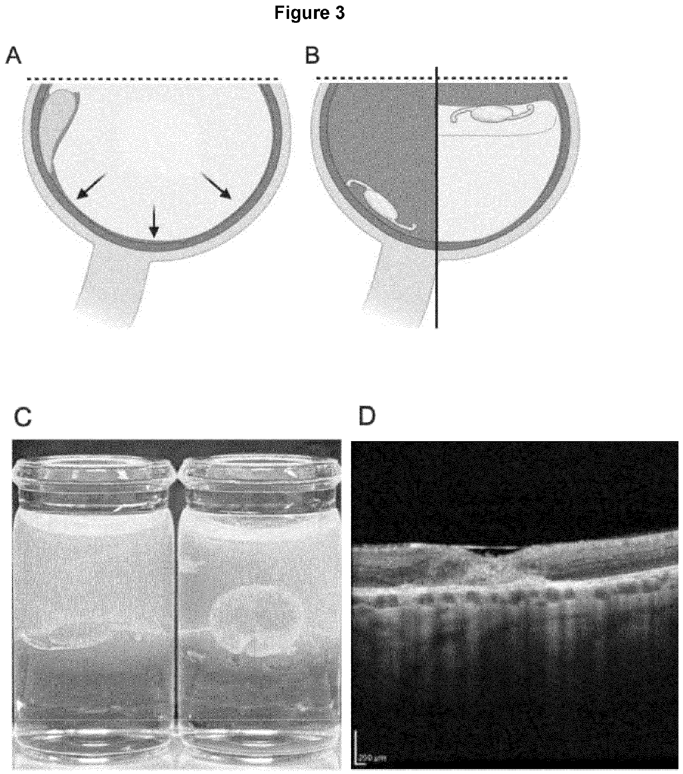

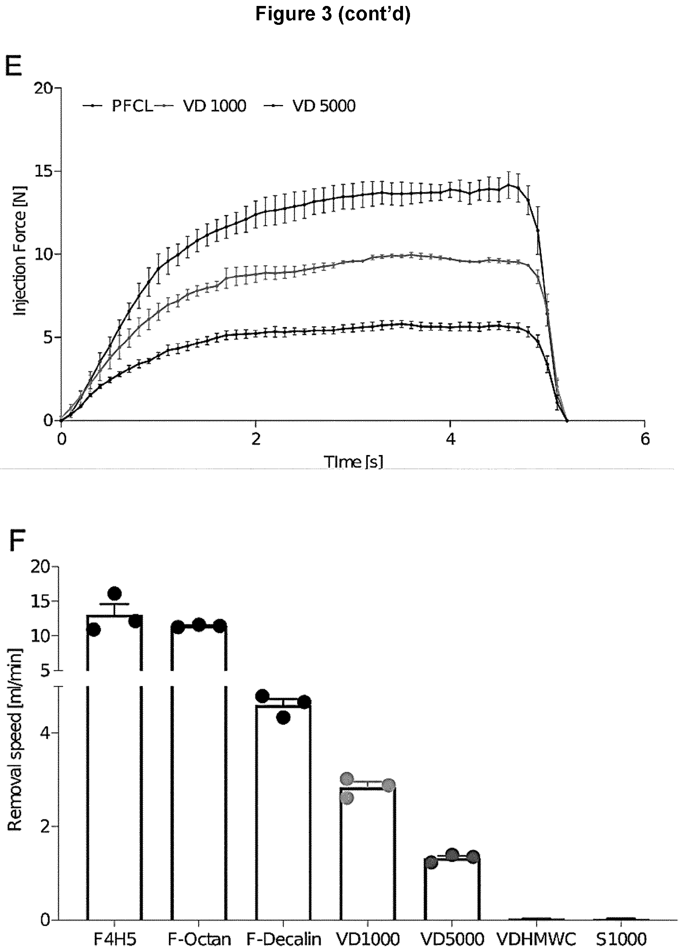

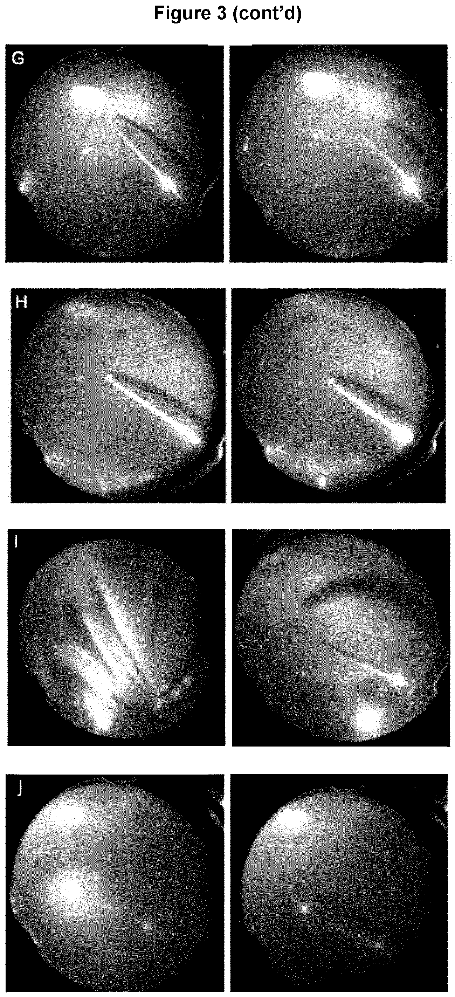

**Fig. 3** shows a graphical representation of the results of assays for determining functioning of a composition according to an embodiment of the present invention comprising 80% w/w perfluorobutylpentane (F4H5), and 20% w/w polydimethylsiloxane (PDMS) as both an intraoperative heavy liquid and postoperative endotamponade. **Fig. 3A** graphically illustrates the main function of an intraoperative heavy liquid being to evacuate subretinal fluid. **Fig. 3B** graphically illustrates a further main function of an intraoperative heavy liquid being to lift foreign bodies away from the posterior pole to protect the retina. **Fig. 3C** shows a photographic representation of an intraocular lense and a crystalline lense, respectively, at the interface of a composition according to an embodiment of the present invention comprising 80% w/w perfluorobutylpentane (F4H5), and 20% w/w polydimethylsiloxane (PDMS) and a stained balanced salt solution (BSS). **Fig. 3D** shows an optical coherence tomography B-scan of a pig 2 weeks after inducing an iatrogenic retinal detachment followed by a tamponade according to an embodiment of the present invention, comprising 80% w/w perfluorobutylpentane (F4H5), and 20% w/w polydimethylsiloxane (PDMS). **Fig. 3E** shows the injection force of compositions according to an embodiment of the present invention comprising 80% w/w perfluorobutylpentane (F4H5), and 20% w/w polydimethylsiloxane (PDMS) in comparison with the injection force of commonly known perfluorocarbon heavy liquids (PFCL). **Fig. 3F** shows the removal speed of compositions according to an embodiment of the present invention comprising 80% w/w perfluorobutylpentane (F4H5), and 20% w/w polydimethylsiloxane (PDMS) in comparison with the removal speed of current clinically used endotamponades. **Fig 3G** shows a photographic representation of a vitreous cavity of a pig wherein a composition according to an embodiment of the present invention comprising 80% w/w perfluorobutylpentane (F4H5), and 20% w/w polydimethylsiloxane (PDMS) is injected by hand. **Fig 3** **H** shows a photographic representation of a vitreous cavity of a pig wherein a composition according to an embodiment of the present invention comprising 80% w/w perfluorobutylpentane (F4H5), and 20% w/w polydimethylsiloxane (PDMS) is removed with a standard 23G-cutter. **Fig 3I** shows a photographic representation of a vitreous cavity of a pig wherein a composition according to an exemplary embodiment of the present invention comprising 80% w/w perfluorobutylpentane (F4H5), and 20% w/w polydimethylsiloxane (PDMS) effectively reattaches the neuroretina. **Fig 3J** shows a photographic representation of a vitreous cavity of a pig wherein an endolaser treatment is performed during a fill with a composition according to an embodiment of the present invention comprising 80% w/w perfluorobutylpentane (F4H5), and 20% w/w polydimethylsiloxane (PDMS).

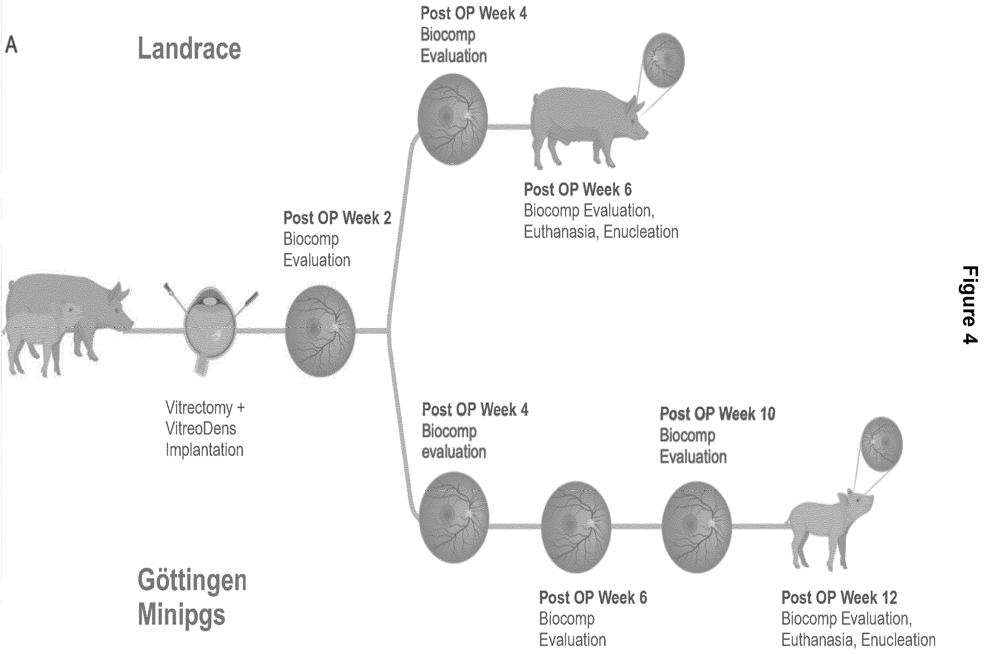

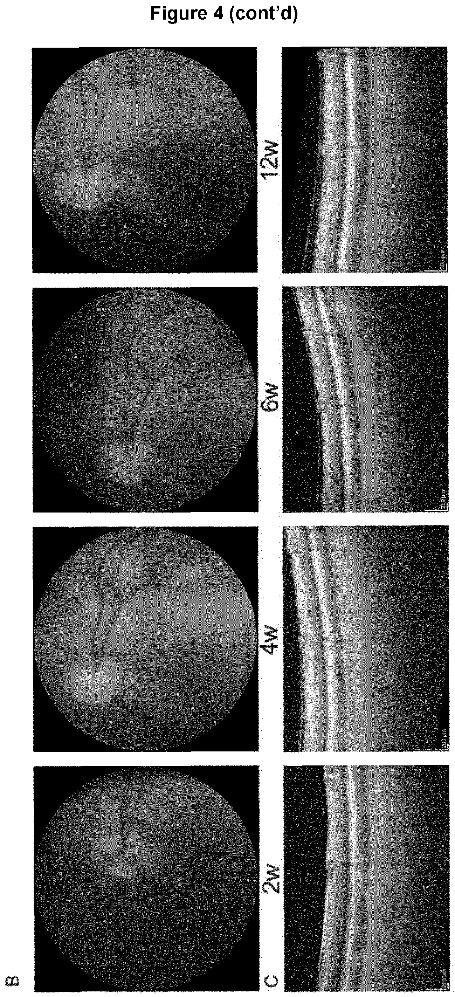

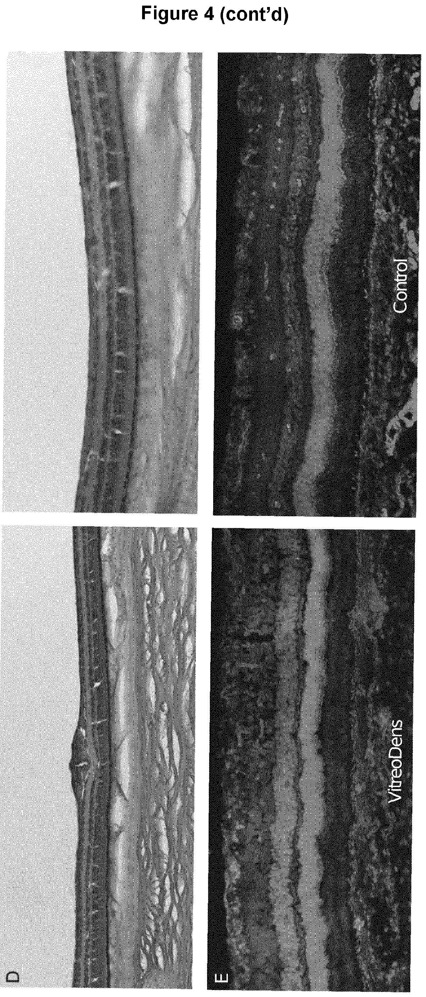

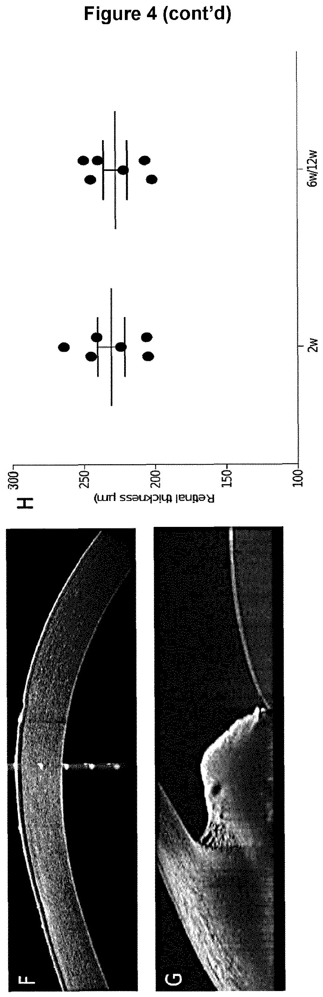

**Fig. 4** shows a graphical representation of the results of assays for determining biocompatibility of a composition according to an embodiment of the present invention comprising 80% w/w perfluorobutylpentane (F4H5), and 20% w/w polydimethylsiloxane (PDMS). **Fig. 4A** graphically illustrates the conducted in-vivo assay for determining biocompatibility. **Fig. 4B** shows fundus photographs of tested animals. **Fig 4C** shows optical coherence tomography images of treated animals, **Fig 4D** shows photographs of a hematoxylin and eosin stain of the retina of treated animals in cross section. **Fig 4E** show photographs of immunostained retina of tested animals in cross section. **Fig 4F** shows an optical coherence tomography image of a treated animal. **Fig 4G** shows an image of the anterior chamber angle of a treated animal. **Fig 4H** shows a graphical representation of retinal thickness measurements of treated animals.

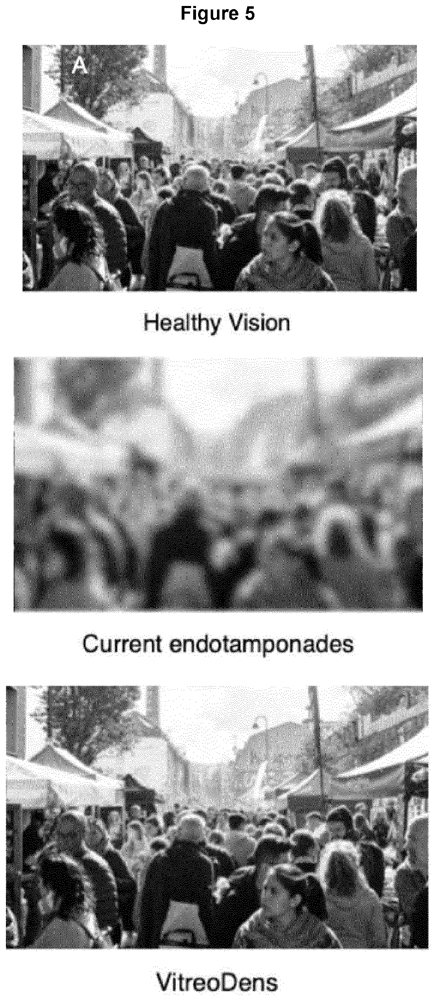

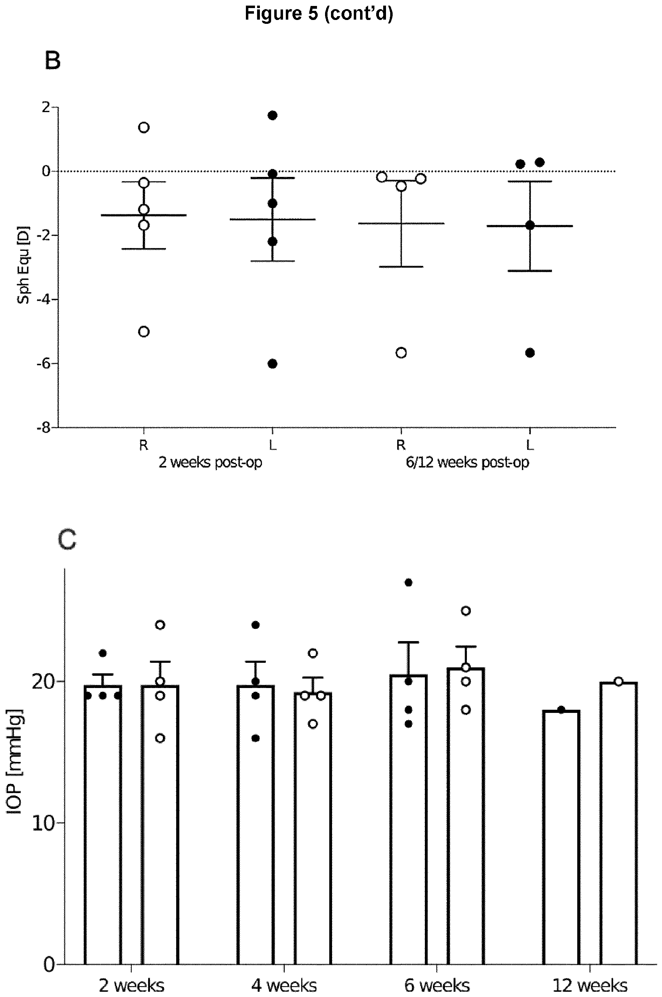

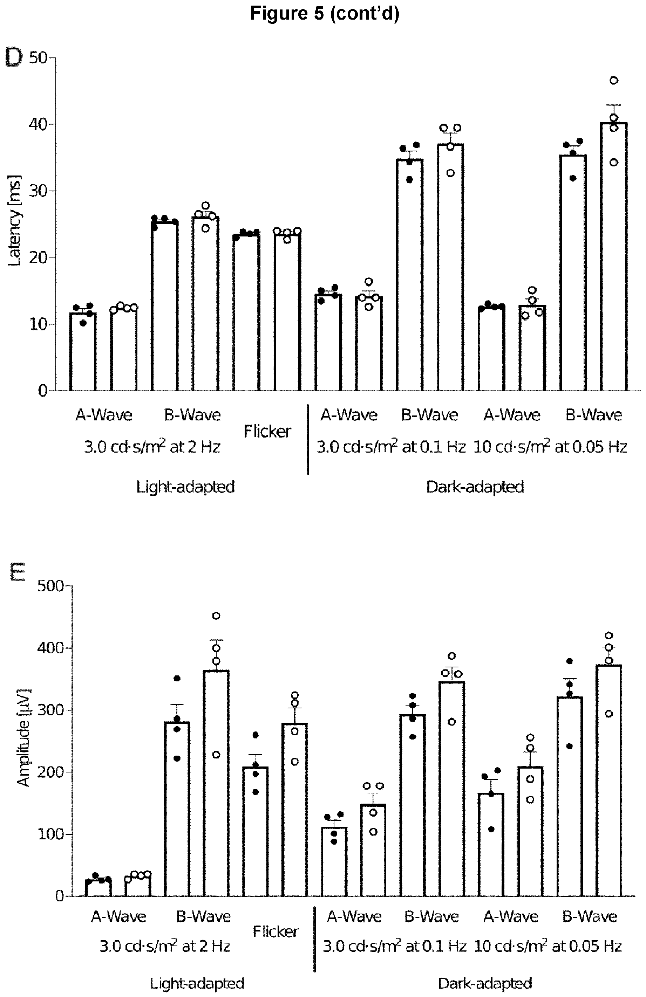

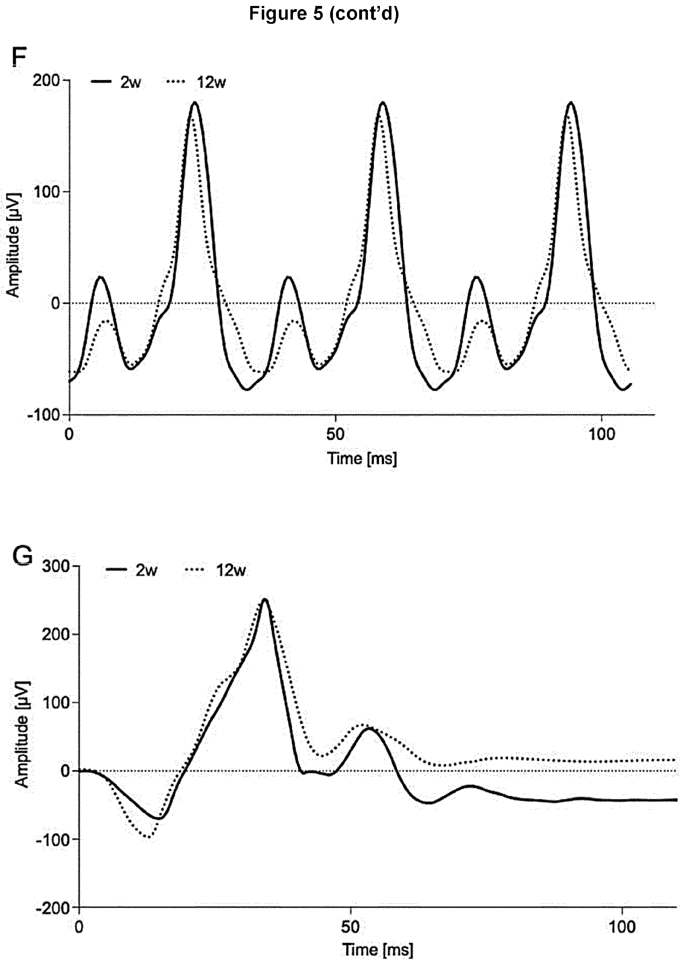

**Fig. 5** shows a further graphical representation of the results of assays for determining biocompatibility of a composition according to an embodiment of the present invention comprising 80% w/w perfluorobutylpentane (F4H5), and 20% w/w polydimethylsiloxane (PDMS). **Fig. 5A** graphically illustrates the refractive shift induced by current endotamponades having a non-optimized refractive index not according to the present invention in comparison to a composition according to the present invention. **Fig 5B** shows a graphical representation of the results of measurements of the Spherical Equivalent (SpH Equ [D]) in treated and non-treated eyes of animals. **Fig 5C** shows a graphical representation of the results of measurements of the intraocular pressure in eyes of animals during treatment. **Fig 5D** shows a graphical representation of an electroretinography depicting the A- and B-wave latency in treated animals. **Fig 5E** shows a graphical representation of an electroretinography depicting the A- and B-wave amplitude in treated animals. **Fig. 5F** shows a graphical representation of light adapted exemplary electroretinographical responses of a treated animal. **Fig. 5G** shows a graphical representation of dark adapted exemplary electroretinographical responses of a treated animal.

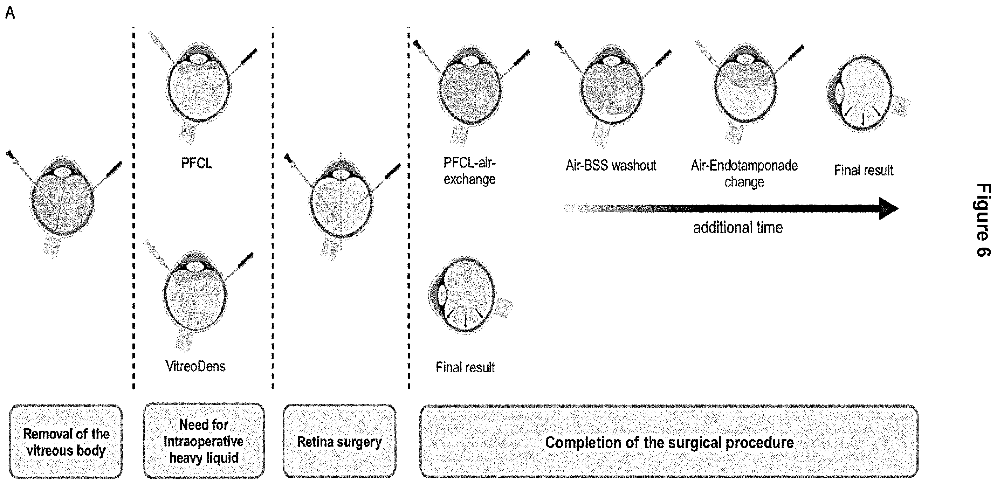

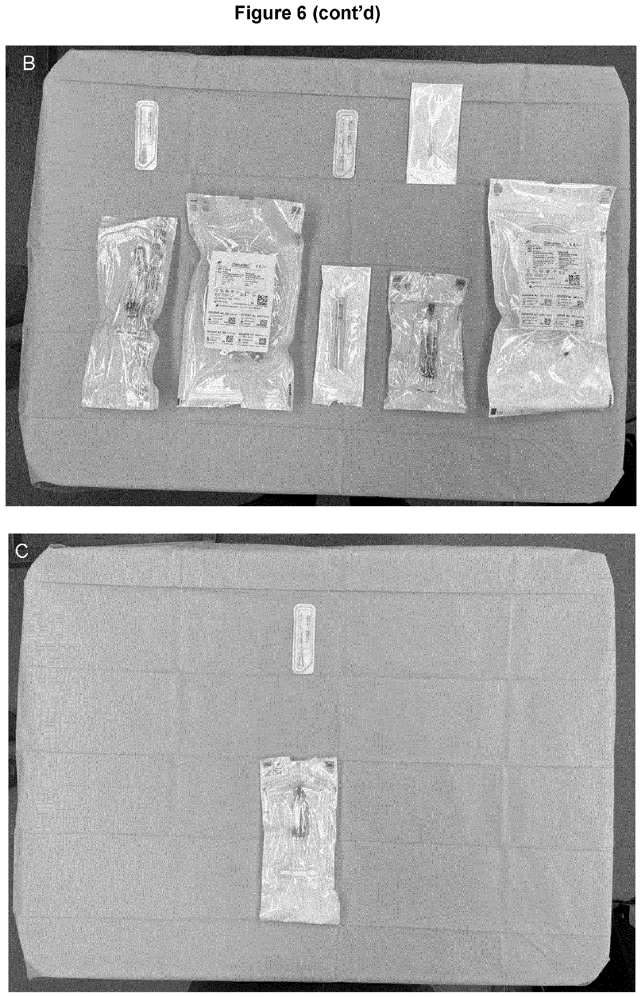

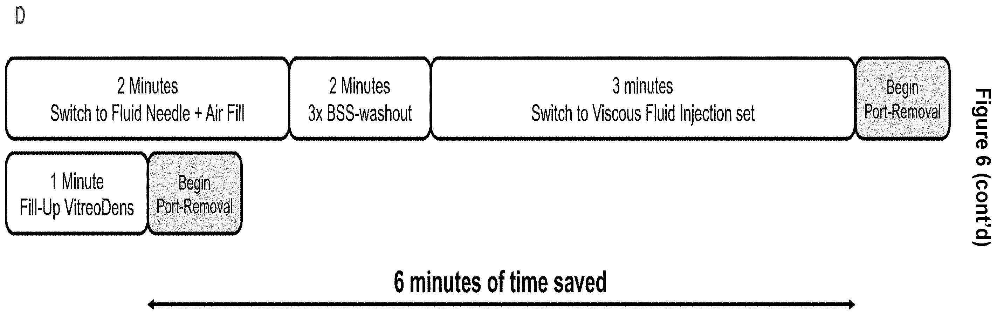

**Fig. 6** shows a graphical representation of the results of assays for determining surgical parameters of a composition according to the present invention. **Fig. 6A** graphically illustrates necessary steps of treatment of an eye with a prior art endotamponade compared with treatment of an eye with a composition according to the present invention. **Fig 6B** shows a photography of surgical waste generated during the surgical procedure using commonly known endotamponades. **Fig 6C** shows a photography of surgical waste generated during the surgical procedure using composition according to the present invention. **Fig. 6D** graphically illustrates the time needed for treatment of an eye with a prior art endotamponade compared with the time needed for treatment of an eye with a composition according to the present invention.

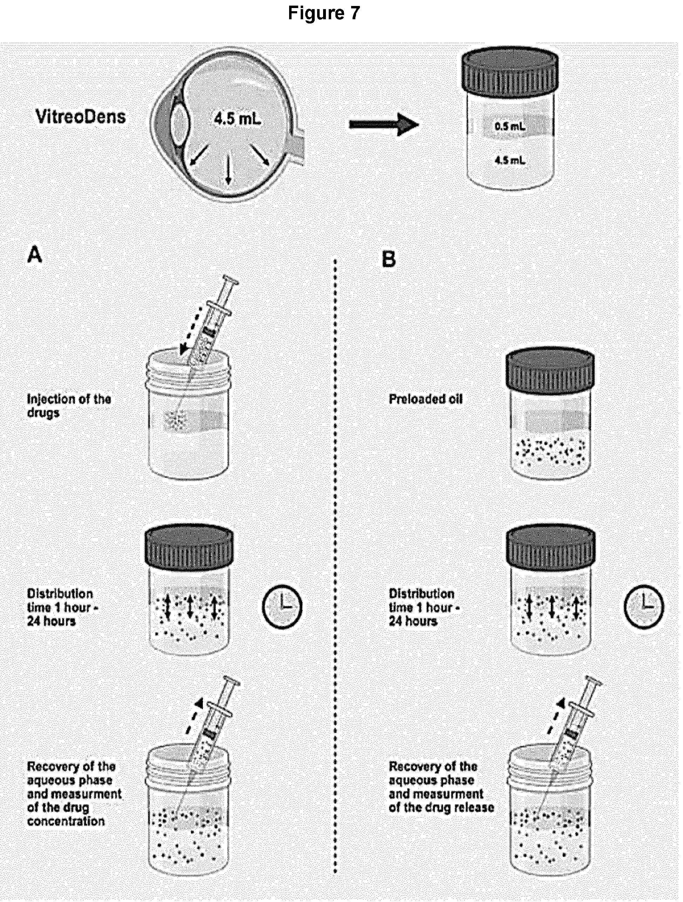

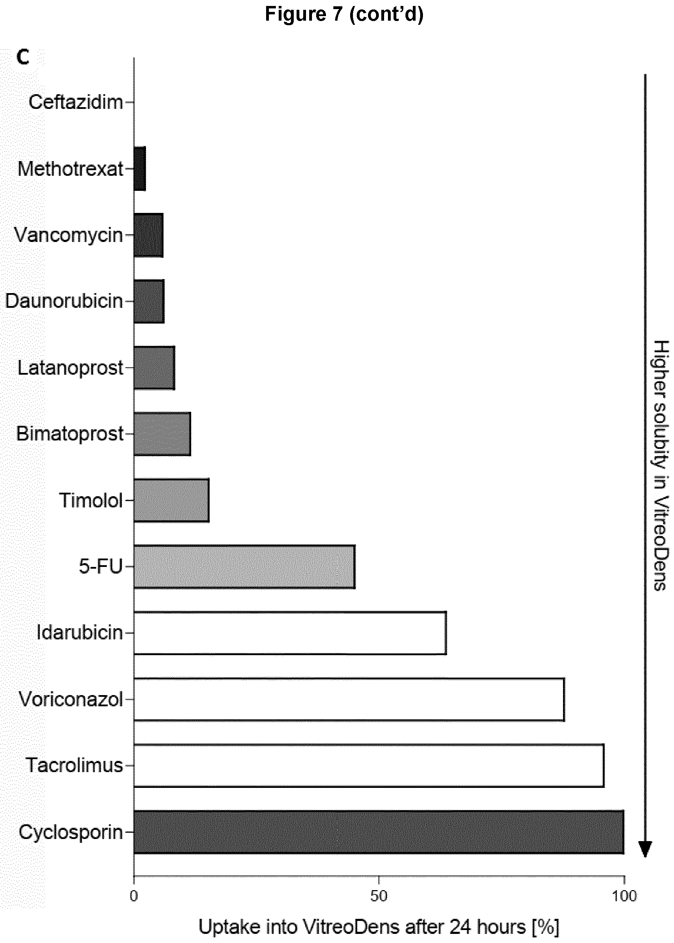

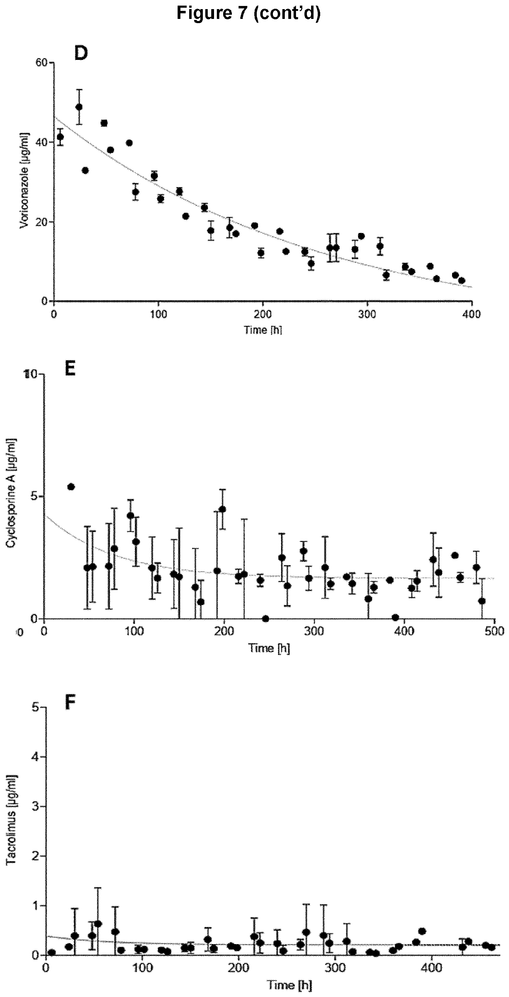

**Fig.** 7 shows a graphical representation of the results of assays for determining drug-uptake and drug-release properties of a composition according to an embodiment of the present invention comprising 80% *w*/*w* perfluorobutylpentane (F4H5), and 20% *w*/*w* polydimethylsiloxane (PDMS). **Fig 7A** and **Fig 7B** graphically illustrate the principles of assays for assessing drug-uptake and drug-release properties. **Fig.7C** shows a graphical representation of the results of solubility measurements of a variety of drugs in a composition according to an embodiment of the present invention comprising 80% *w*/*w* perfluorobutylpentane (F4H5), and 20% *w*/*w* polydimethylsiloxane (PDMS). **Fig 7D** shows a graphical representation of the results of an assay for determing the voriconazole release time of a composition according to an embodiment of the present invention comprising 80% *w*/*w* perfluorobutylpentane (F4H5), and 20% *w*/*w* polydimethylsiloxane (PDMS). **Fig 7E** shows a graphical representation of the results of an assay for determing the cyclosporine A release time of a composition according to an embodiment of the present invention comprising 80% *w*/*w* perfluorobutylpentane (F4H5), and 20% *w*/*w* polydimethylsiloxane (PDMS). **Fig 7F** shows a graphical representation of the results of an assay for determing the Tacrolismus release time of a composition according to an embodiment of the present invention comprising 80% *w*/*w* perfluorobutylpentane (F4H5), and 20% *w*/*w* polydimethylsiloxane (PDMS).

### DETAILED DESCRIPTION OF THE INVENTION

As explained above, in a first aspect the invention is directed to a (e.g. intraocular endotamponade) composition comprising a mixture of at least one oil and one or more semifluorinated alkanes, the refractive index of the composition being from about 1.310 to about 1.365.

Methods for measuring a refractive index of an optical medium are known in the art. For example, the "refractive index" of an optical medium as herein referred to may be measured according to the method described in Example 1 and corresponding materials and methods. In particular, all values for a refractive index as described herein refer to a refractive index measured at a temperature of 35 °C. It is believed that the values obtained by measuring the refractive index at 35 °C more closely reflect the natural situation in the human eye, as e.g. a mid-vitreous temperature has been reported to be 34.8 ± 0.9 °C [Shinoda K et al.; J. Clin. Med. 2021, 10, 3412]. All values for a refractive index as herein described relate to values obtained by standard refractive index measurements taken at the "yellow doublet" sodium D line, with a wavelength (λ) of 589 nanometers.

By providing a (e.g. intraocular endotamponade) composition comprising at least one oil and one or more semifluorinated alkanes, the refractive index of the composition being from about 1.310 to about 1.365, the inventors could surprisingly show that it is possible to provide compositions suitable to be used as tamponade composition wherein the refractive index of the composition is closer to -optionally even identical with -a refractory index of the natural vitreous body of the eye than refractive indexes of prior art tamponade agents comprising silicone oils. For example, typical silicon oil tamponades known in the art have a refractory index of 1.4 [Vaziri K. et al. Clinical Ophthalmology 2016:10].

As the human vitreous is composed of over 98% water, the refractory index of the "natural vitreous body" as referred to herein is comparable with / almost identical with the refractory index of water, being 1.331 at 35 °C.

According to an embodiment, the refractive index of the composition may be from about 1.315 to about 1.360, such as from about 1.320 to about 1.350, preferably wherein the refractive index may be from about 1.325 to about 1.345, such as from about 1.330 to about 1.340, most preferably wherein the refractive index may be about 1.331.

According to an embodiment, the refractive index of the composition may be identical with or close to the refractory index of the natural human vitreous body. A refractory index of the composition may be "close to" the refractory index of the natural human body if the refractory index of the composition is within +/-0.005, +/- 0.004, +/- 0.003, +/- 0.002 or +/- 0.001 dimensionless number units from the refractory index of the natural human body.

The inventors have found that the closer the refractive index of the composition approaches the refractive index of the natural vitreous body, the more the above described problems for patients during the time of use of the tamponade may be reduced/avoided. For example, patients may no longer need strong correction of visual acuity during the time of application of the tamponade. If the refractive index of the composition is about 1.331 or close to this value, e.g. with respect to patients who normally do not need any correction of visual acuity, a correction of visual acuity may no longer be necessary.

For example, the refractive index of the composition may be from about 1.310 to about 1.360, from about 1.310 to about 1.355, from about 1.310 to about 1.350, from about 1.310 to about 1.345, from about 1.310 to about 1.340, from about 1.310 to about 1.335, from about 1.315 to about 1.360, from about 1.315 to about 1.355, from about 1.315 to about 1.350, from about 1.315 to about 1.345, from about 1.315 to about 1.340, from about 1.315 to about 1.335, from about 1.320 to about 1.360, from about 1.320 to about 1.355, from about 1.320 to about 1.350, from about 1.320 to about 1.345, from about 1.320 to about 1.340, from about 1.320 to about 1.335, from about 1.325 to about 1.360, from about 1.325 to about 1.355, from about 1.325 to about 1.350, from about 1.325 to about 1.345, from about 1.325 to about 1.340, from about 1.325 to about 1.335, from about 1.330 to about 1.360, from about 1.330 to about 1.355, from about 1.330 to about 1.350, from about 1.330 to about 1.345, from about 1.330 to about 1.340, from about 1.330 to about 1.335, or from about 1.330 to about 1.332.

According to an embodiment, the one or more semifluorinated alkane my comprise perfluorobutylpentane (F4H5), perfluorobutylhexane (F4H6), perfluorobutyloctane (F4H8), perfluorohexylhexane (F6H6), perfluorohexyloctane (F6H8) or a mixture thereof. Preferably the semifluorinated alkane may be perfluorobutylpentane (F4H5).

In accordance with an embodiment, the at least one oil may be selected from one or more of the group consisting of a silicone oil, a fluorosilicone oil, a silicone-fluorosilicone copolymer oil, a silicone-phenylsilicone copolymer oil, a silicone oil derivative and a mixture thereof. Preferably, at least one oil may be a silicone oil. The silicone oil may be a polydimethylsiloxane.

According to the invention, flowable silicones, in particular siloxanes, are preferred as silicone oils. Methylsiloxanes and their polymers and dimethylsiloxanes and their polymers are well suited for use in the compositions of the invention. The silicones can be both unsubstituted and substituted. Substitution is the process of replacing a hydrogen atom bound to a silicon atom with another atom or group of molecules. Common substituents are straight-chain, branched, or cyclic alkyl groups with 1 to 18 carbon atoms or aryl groups with 6 to 15 carbon atoms. The alkyl or aryl groups may be self-substituted. The substituents may be in blocks or distributed over the siloxane chain and their quantity may be adjusted in a known way according to the desired properties.

Examples of silicone oils also include, but are not limited to: n-polydimethylsiloxane, iso-polydimethylsiloxane, aryl-substituted PDMS, such as phenyl-polydimethylsiloxane, diphenyl-polydimethylsiloxane, polyphenyl-polydimethylsiloxane, alkyl-substituted PDMS, such as methyl-polydimethylsiloxane, ethyl-polydimethylsiloxane, propyl-polydimethylsiloxane, and the like, PDMS with other substituents, such as fluoroalkyl-substituted PDMS. Examples of other suitable silicone oils are bisdiphenylethyldisiloxane, bisphenylhexamethicone, capryldimethicone, caprylyldimethicone, caprylylmethicone, dimethicone, disiloxane, hexyldimethicone, hexylmethicone, lauryldimethicone, lauryl methicone, methicon, methyltrimethicone, phenylethyldimethicone, phenylethyldisiloxane B, phenylpropylethylmethicone, phenylpropyltrimethicone, phenyltrimethicone, trifluoropropyldimethicone, trifluoropropyl methicone, trisiloxane, etc.

Advantageously, the one or more semifluorinated alkane (e.g. F4H5) and the at least one oil (e.g. at least one silicone oil such as e.g.at least one polydimethylsiloxane) are miscible with each other in any mixing ratio. These characteristics may allow for the provision of a composition having the refractive index according to the present invention.

As explained above, the composition of the present invention comprises a mixture of at least one oil in addition to one or more semifluorinated alkanes. Apart from one or more semifluorinated alkanes, other additives may also be present in the compositions of the invention. The final composition has 100 wt. %.

It is envisaged that the one or more semifluorinated alkanes may be present in the composition in a quantity in a range of about 56% w/w to about 99% w/w, or of about 70% w/w- to about 90 % w/w, or of about 75.1 % w/w to about 90% w/w, based on the total weight of the composition. Preferably, the one or more semifluorinated alkanes may be present in the composition in a quantity in a range of about 77% w/w to about 85% w/w, based on the total weight of the composition.

For example, one or more semifluorinated alkane(s) can be found in an amount of about 56 wt. %, 60 wt. %, 65 wt. %, 70 wt. %, 75 wt. %, 77 wt. %, 78 wt. %, 79 wt. %, 80 wt. %, 81 wt. %, 82 wt. %, 83 wt. %, 84 wt. %, 85 wt. %, 87 wt. %, 90 wt. %, 92 wt. %, 94 wt. %, 96 wt. %, 98 wt. or 99 wt. % in relation to the entire composition (such as intraocular endotamponade) (100% by weight). One or more semi-fluorinated alkane(s) can optionally be included in an amount in the range of about 56 wt, % - 99 wt. %, 56 wt. % - 90 wt. %, 56 wt. % - 85 wt. %, 70 wt. % - 99 wt. %, 70 wt. % - 90 wt. %, 70 wt. % - 85 wt. %, 75.1 wt.% - 99 wt.%, 75.1 wt.%-90 wt.%, 75.1 wt.% - 85 wt.%, 77 wt.% - 99 wt.%, 77 wt.% - 90 wt.%, or 77 wt.% - 85 wt.%, in relation to the entire composition, (such as the intraocular endotamponade) (100% by weight).

A composition of the present invention may consist of (x) wt% of at least one oil and (z)% wt/wt. of one or more semifluorinated alkanes. If, for example, one or more semifluorinated alkanes are present in the composition in the range of about 77 to 90% by weight, the at least one (silicone) oil is within the range of about 10 to 23% by weight. If, however, the composition of the present invention comprises another compound in addition to one or more semifluorinated alkanes (z% w/w) and the at least one oil (x) % w/w), then either the amount of one or more semifluorinated alkanes and/or the amount of the at least one oil can be reduced accordingly, so that the composition is 100% wt/wt. (100 w/w). Accordingly, either the amount of one or more semifluorinated alkanes or the amount of the at least one oil can be reduced, or both the amount of one or more semifluorinated alkanes and the amount of the at least one oil can be reduced.

According to an embodiment, the at least one oil (e.g. the at least one silicone oil) may be present in the composition in a quantity in a range of about 1% w/w to about 44% w/w, or of about 10% w/w to about 30% w/w, or of about 10% w/w to about 24.9 % w/w, based on the total weight of the composition. Preferably the at least one oil may be present in the composition in a quantity in a range of about 15% w/w to about 23% w/w, based on the total weight of the composition.

For example, the at least one oil (e.g. the at least one silicone oil) may be present of about 1 wt. %, 2wt. %, 4 wt. %, 6 wt. %, 8 wt. %, 10 wt. %, 13 wt. %, 15 wt. %, 16 wt. %, 17 wt. %, 18 wt. %, 19 wt. %, 20 wt. %, 21 wt. %, 22 wt. %, 23 wt. %, 25 wt. %, 30 wt. %, 35 wt. %, 40 wt. %, or 44 wt. % in relation to the entire composition (such as intraocular endotamponade) (100% by weight). One or more oil (e.g. at least one silicone oil) may optionally be included in an amount in the range of about 1 wt, % - 44 wt. %, 1 wt. % - 30 wt. %, 1 wt. % - 24.9 wt. %, 1 wt. % - 23 wt. %, 10 wt. % - 44 wt. %, 10 wt. % - 30 wt. %, 10 wt.% - 24.9 wt.%, 10 wt.% - 23 wt. %, 15 wt.% - 44 wt.%, 15 wt.% - 30 wt.%, 15 wt.% - 24.9 wt.%, or 15 wt.% - 23 wt.%, in relation to the entire composition, (such as the intraocular endotamponade) (100% by weight).

According to an embodiment, the at least one oil may comprise an oil having a viscosity of about 9,999 mPas or less, such as e.g. of about 7,000 mPas or less; and/or the at least one oil may comprise an oil having a viscosity in the range of about 10,000-25,000,000 mPas.

For example, the at least one oil may comprise an oil having a viscosity of about 1,000 mPas, and/or of about 2,000 mPas, and/or of about 5,000 mPas.

The term 'elongational viscosity', as used herein, has the unit pascalsecond (Pa·s) and is a measure of the resistance of a substance to flow in a strain flow. What elongational viscosity means can be understood by considering a dilute solution of a linear polymer with high molecular weight, where the properties of the solution are determined by the isolated individual polymer coils. At low expansion rates of the solution, the polymer chains are in a loosely spherical configuration and the elongational viscosity is low. However, at higher expansion rates, the polymer chains unwind and elongate in line with the direction of expansion, presenting a resistance to the applied extensional flow. At a critical strain flow rate, the polymer chains unwind into an elongated cord. This is known as the coil-stretch transition and results in a large increase in resistance to the applied expansion and thus an elongational viscosity that can be many times higher than the equivalent viscosity in the shear flow.

How the elongational viscosity is measured is known to the specialist. For example, the elongational viscosity can be measured with an elongational rheometer. Preferably, this measurement is carried out at room temperature, i.e. at a temperature of 19-25 °C, preferably at 25 °C.

The process of emulsification may be construed as the oscillation of the shear of an oil/water interface under an external force that leads to the pulling out of filaments of one liquid into another. These filaments, which subsequently become thin as they expand, then break up, resulting in the formation of satellite droplets that, if suitable emulsifying stabilizers are present, remain in the continuous phase. By increasing the elongation viscosity of the oil phase, filament breakage is inhibited and therefore an increase in elongation viscosity prevents the formation of droplets.

According to an embodiment, the composition may comprise (or consist of) about 80% w/w perfluorobutylpentane (F4H5), and about 20% w/w polydimethylsiloxane (PDMS), based on the total weight of the composition. According to some embodiments, the viscosity of the composition may be between 7 mPas and 2481 mPas. For example, the viscosity of the composition may be set by varying the molecular weight of the PDMS. For example, in a composition comprising (or consisting of) about 80% w/w perfluorobutylpentane (F4H5), and about 20% w/w polydimethylsiloxane (PDMS), the viscosity of the PDMS may be about 1000mPas, about 5000 mPas, or about 2.5 million mPas.

According to a further embodiment, the at least one oil may comprise at least a first oil and a second oil, wherein the first oil may have a viscosity of about 9,999 mPas or less, such as of about 7,000 mPas or less, and further wherein the second oil may have a viscosity in the range of about 10,000-25,000,000 mPas,

Alternatively, the first oil and the second oil may both have a viscosity about 9,999 mPas or less, such as about 7,000 mPas or less.

Alternatively, first oil and the second oil may both have a viscosity in the range of about 10,000-25,000,000 mPas.

If the first oil used in the composition according to this embodiment is a low viscosity oil such as oils currently used in procedures for the treatment of retinal detachment, and if the second oil has a high molecular weight and will be soluble/miscible with the first oil, physical properties, like extensional viscosity, shear viscosity, emulsion tendency, refraction index, density and biocompatibility of the composition may be advantageously affected. It will be apparent to one skilled in the art that the quantity of the second oil relative to the quantity of the first oil will depend upon a number of factors, not least the particular molecular weights and/or viscosities of the first and the second oil. For example, a small quantity of a larger molecular weight second oil may produce the same increase in extensional viscosity of the first oil as a larger quantity of a smaller molecular weight second oil. It may be preferable that the quantity of second oil is relatively low in the composition in relation to the quantity of the first oil so that the additive does not detrimentally affect the shear viscosity of the oil. Controlling the shear viscosity of the oil is important so that the composition can be inserted into the eye using the smallest incision as possible.

For example, the first oil may be present in the composition in a quantity in the range of about 1% w/w - about 43 % w/w, and the second oil may be present in the composition in a quantity in a range of about 1% w/w - about 43 % w/w, based on the total weight of the composition.

According to an embodiment, both the first and the second oil may be selected from one or more of the group consisting of a silicone oil, a fluorosilicone oil, a silicone-fluorosilicone copolymer oil, a silicone-phenylsilicone copolymer oil, a silicone oil derivative and a mixture thereof. Preferably, both the first and the second oil may be a silicone oil. The silicone oil may be a polydimethylsiloxane.

It is further envisaged that the at least one oil comprises additional oil(s) further to the e.g. first and second oil as herein described. Further oils may also be selected from one or more of the following: a silicone oil, a fluorosilicone oil, a silicone-fluorosilicone copolymer oil, a silicone-phenylsilicone copolymer oil, a silicone oil derivative, or a mixture thereof.

According to an embodiment, the composition further comprises additives selected from one or more of emulsification inhibitors, steroids, non-steroidal anti-inflammatory agents, antibiotics, or a mixture thereof.

It is herewith contemplated that the composition may comprise additives like emulsification inhibitors (e.g. hydrophobic additives or detergents) or pharmacologically active agents, like steroids e.g. corticosteroids like glucocorticoids (e.g. dexamethasone), fluocinolone acetonide or triamcinolone acetonide; other non-steroidal anti-inflammatory agents like acetylsalicylic acid, phenylbutazone or nabumetone; or antibiotics like vancomycin and ceftazidime; or antifungal agents like voriconazole but not limited thereon.

For example, the additive may be present in the composition in a quantity in a range of about 0.0001 to about 10 % w/w, optionally from about 0.1 to about 5 % w/w, based on the weight of the total composition.

Reverting to the aspects of the invention, in a further aspect the invention is directed to the composition according to the first aspect for use in treating a disease.

For example, the use may comprise temporary treatment for the posterior and/or anterior segment of the eye.

In accordance with an embodiment, the disease may be a detached retina.

It is envisaged that the composition may be used intraoperable, as a short-term tamponade, as a mid-term tamponade, or as a long-term tamponade.

"Intraoperative" as referred to herein may relate to an application period from about 1 minute to about 180 minutes. "Short-term tamponade" as referred to herein may refer to an application period from about 180 minutes to about 30 days. "Mid term tamponade" as referred to herein may relate to an application period from about 31 days to about 60 days. "Long term tamponade" as referred to herein may relate to an application period from about 61 days to about 365 days and longer.

In yet a further aspect the inventon relates to a kit of parts comprising the composition as herein provided in a vial or syringe, one or more of cannulas and/or tubing, and instructions for use.

According to a further aspect, there is provided a method of producing the composition as herein provided for use in the treatment of a disease such as e.g. a detached retina, comprising mixing at least one oil and one or more semifluorinated alkanes to produce a composition as herein provided.

In a further aspect, the invention relates to a method for treating a disease in a subject, optionally comprising treating retinal detachment in a subject in need thereof, comprising administering the composition as herein provided into the vitreous body of the eye of said subject.

According to a further aspect, there is also provided a method for treating retinal detachment in a subject in need thereof, the subject further suffering from ametropia, comprising administering a composition into the vitreous of the eye of said subject; wherein the composition comprises a mixture of at least one oil in a first quantity and one or more semifluorinated alkanes in a second quantity, and further wherein the method comprises the step of selecting the first quantity of the at least one oil and the second quantity of the one or more semifluorinated alkanes, thereby adjusting a refractive index of the mixture such that the administered composition corrects the ametropia of the subject.

By the above method, by purposively selecting the refractive index of the composition depending on an existing level of ametropia in the patient, advantageously, patients which normally are in need of a correction of visual acuity may, during the duration of the treatment with the (tamponade) composition, no longer need a correction of visual acuity by e.g. glasses.

For example, the one or more semifluorinated alkan may comprise perfluorobutylpentane (F4H5), perfluorobutylhexan (F4H6), perfluorobutyloctane (F4H8), perfluorohexylhexane (F6H6), perfluorohexyloctane (F6H8) or a mixture thereof; preferably the semifluorinated alkane being perfluorobutylpentane (F4H5), and/or the at least one oil may be selected from one or more of the group consisting of a silicone oil, a fluorosilicone oil, a silicone-fluorosilicone copolymer oil, a silicone-phenylsilicone copolymer oil, a silicone oil derivative and a mixture thereof, preferably wherein the at least one oil is a silicone oil.

According to an embodiment, the composition of the method may further comprise additives selected from one or more of emulsification inhibitors, steroids, non-steroidal anti-inflammatory agents, antibiotics or a mixture thereof.

The invention will be further illustrated by the following non-limiting Experimental Examples.

### Experimental Examples

### Materials and Methods:

Measurements of the refractive index:
The refractive index of optical mediums was measured using a NOVEX ABBE REFRAKTOMETER 98.490. Before commissioning, the refractometer was tempered for at least 20 minutes and before each measurement, it was checked that the set temperature is displayed on the built-in thermometer before each measurement. For the measurements performed in the Examples as herein presented, the temperature for each measurement was set to be 35°C. General calibration was performed in accordance with the calibration protocol for the refractometer.

Before each measurement, the correct calibration of the refractometer was re-checked with distilled water (nD35: 1.33131 = set point 35°C) to make sure that the thermometer is intact. It was verified that the reading did not deviate by more than 0.0005 from the nominal value. A new calibration was carried out if the check with distilled water was not successful. Each measurement is repeated three times, and the mean value is calculated.

For mixing the solutions, Siluron^{®} 1000, Siluron^{®} 2000, Siluron^{®} 5000 and F4H5^{®} were provided by Fluoron GmbH, high-molecular-weight component (HMWC) polydimethylsiloxane (PDMS) was provided by Gelest.

### Example 1: Mixture A

A solution is prepared by mixing 10% w/w high-molecular-weight component (HMWC) polydimethylsiloxane (PDMS) with 90% w/w F4H5. The refractive index of mixture A is measured three times.

### Example 2: Mixture B

A solution is prepared by mixing 10% w/w Siluron^{®} 1000 with 90% w/w F4H5. The refractive index of mixture B is measured three times.

### Example 3: Mixture C

A solution is prepared by mixing 25% w/w Siluron^{®} 1000 with 75% w/w F4H5. The refractive index of mixture C is measured three times.

### Example 4: Mixture D

A solution is prepared by mixing 15% w/w Siluron^{®} 5000 with 15% w/w high-molecular-weight component (HMWC) polydimethylsiloxane (PDMS) and with 70% w/w F4H5. The refractive index of mixture D is measured three times.

### Example 5: Mixture E

A solution is prepared by mixing 40% w/w Siluron^{®} 5000 with 10% w/w high-molecular-weight component (HMWC) polydimethylsiloxane (PDMS) and with 50% w/w F4H5. The refractive index of mixture D is measured three times.

### Example 6: Mixture F

A solution is prepared by mixing 69.5% w/w Siluron^{®} 5000 with 30.5% w/w F4H5. The refractive index of mixture F is measured three times.

### Example 7: Mixture G

A solution is prepared by mixing 62.55% w/w Siluron^{®} 1000 with 6.95% w/w **high-molecular-weight** component (HMWC) polydimethylsiloxane (PDMS) and with 30.5% w/w F4H5. The refractive index of mixture G is measured three times.

### Synthesis and characterization of PDMS-F4H5 compositions comprising 80% w/w perfluorobutylpentane (F4H5), and 20% w/w polydimethylsiloxane (PDMS):

A variety of mixtures with varying F4H5:PDMS ratios were examined for their refractive index and density. After establishing the relationship between the F4H5:PDMS ratio, refractive index and density, PDMS of differing molecular weight (1000, 5000 and 2.5 mio mPas) were used to titrate the desired viscosity, leading to the three lead compositions comprising 80% w/w perfluorobutylpentane (F4H5), and 20% w/w polydimethylsiloxane (PDMS) (VitreoDens-candidates) with the refractive index of water at 35°C.

### Rheology and Interfacial Tension:

The rheometer Anton Paar MCR, 302e (Anton Paar, Graz, Austria) was used for all measurements at 20° C using a cone-plate geometry. An amplitude sweep was conducted from 0.01 to 10 % shear deformity in triplicate for the three primary prototypes. A KRÜSS Spinning Drop Tensiometer (KRÜSS GmbH, Hamburg, Germany) was used to measure the static interfacial tension in a fully automated fashion. The respective prototype was used as the lipophilic phase, balanced salt solution (BVI Medical, Waltham, MA, USA), an electrolyte solution mimicking the intraocular environment, was used as the hydrophilic phase.

### Assessment of emulsification tendency and electrochemical emulsion stability:

Emulsification was induced in line with experiments presented by Caramoy et al.; Br J Ophthalmol 94, 509-512 (2010). In short, the sample and a standardized emulsifier (serum of a healthy 26-year old male) are placed in a cuvette. Emulsification is induced and in immediate succession the cuvette is placed in a centrifuge at 5000 G for 30 minutes (Fig. 2C). After centrifugation, the emulsified area is evaluated using ImageJ. Zeta-potentials were measured using the ZetaSizer Nano ZS (Malvern Panalytical) in triplicate for all compositions and the three lead candidates. To test the emulsion stability, no emulsifiers were used. The zeta potential was measured to assess electrochemical stability of in vitro-generated emulsions (1 ml sample, 9 ml BSS vortexed and afterwards placed in an ultrasound bath for 5 minutes). A higher absolute zeta potential within the same hydrophilic media, would speak for an increase an emulsion stability (Fig. 2A). Results were compared to previous measurements for clinically available silicone oils as published by Hammer et al.; Trans. Vis. Sci. Tech. 11, 3 (2022).

### Forward light scattering, turbidity and transmission spectra:

Turbidity was measured using the TURB430 IR (Xylem Analytics, Weilheim, Germany). Forward light scattering was measured using a previously established in-vitro setup first presented in Labuz et al.; Biomed. Opt. Express 6, 4457 (2015), that was later optimized for the measurements of silicone oils Hammer et al.; Ophthalmology Science, 100558 (2024), and the vitreous body Hammer et al.; Invest. Ophthalmol. Vis. Sci. 65, 36 (2024).

### Sterilization:

10 ml of the composition comprising 80% w/w perfluorobutylpentane (F4H5), and 20% w/w polydimethylsiloxane (PDMS) (VitreoDens) were filled into vials, stomped and crimped. Vials were placed in a steam autoclave for 20 minutes at 121°C. Subsequently, sterility testing was performed based on the European Pharmacopoiea's guideline (Ph. Eur. 2.6.1 (11.5)).

### Endotoxin and Genotoxicity assessments:

Samples of the composition comprising 80% w/w perfluorobutylpentane (F4H5), and 20% w/w polydimethylsiloxane (PDMS) (VitreoDens) were tested for endotoxin content based on two separate European Pharmacopoiea guidelines (Ph. Eur. 2.6.14/2.6.32:2021) using either Limulus Amebocyte Lysate (LAL) or recombinant factor C to also allow the quantification of gram-negative bacteria.

### Purity:

Both components of the final composition comprising 80% w/w perfluorobutylpentane (F4H5), and 20% w/w polydimethylsiloxane (PDMS) (VitreoDens-mixture) were investigated on impurities according to the European Pharmacopeia 2.2.28 (test: "gas chromatography"). This assay confirms the absence of volatile low molecular weight components.

### In-vitro cytotoxicity:

Possible cytotoxic effects were evaluated in vitro in two cell lines: A human retinal pigment epithelium (ARPE-19) cell line and L929, a fibroblast cell line. PrestoBlue^{®} assays were conducted using a direct contact method (method (as described by ISO 10993-5, 2009) and a transwell method as previously established for silicone oils in ARPE-19 cells (Hammer et al.; Retina (2024), doi:10.1097/IAE.0000000000004324). Fig. 2 F depicts the process for both. For the L929 line, a standardized cell growth analysis via BCA-staining with extracts of VitreoDens was performed following internationally accepted guidelines and recommendations for the biological evaluation of medical devices, especially DIN EN ISO 10993-1: 2021 "Evaluation and testing within a risk management process", DIN EN ISO 10993-5: 2009 "Tests for in vitro cytotoxicity" and DIN EN ISO 10993-12: 2021 "Sample preparation and reference materials".

### Injection pressure and Removal Speed:

The resistance force during the injection of each substance was measured and evaluated using an automated digital force gauge meter FV-10XY (Shimpo Instruments, Linbrook, USA) with the Shimpo Toriemon Force Gauge Software. Removal speed was evaluated as previously pubished (Hammer et al.; Int J Retin Vitr 9, 43 (2023)). In short, in-vitro removal speed was measured by applying an active vacuum of 600 mmHg connected to a 23G-cutter (Geuder AG, Heidelberg, Germany) with a Geuder S4 Phaco-Vitrectomy Machine (Geuder AG, Heidelberg, Germany) for a specified amount of time. Samples were weighed before and after and an aspirated volume was calculated and corrected for the density of the removed substance. All measurements were performed in triplicates.

### Surgical evaluation as an intraoperative heavy liquid:

First, the in vitro function as a heavy liquid, namely the ability to lift intraocular lens and remnants of the natural crystalline lens was evaluated. For this, a negatively charged glass container was filled with BSS, then the to be lifted foreign body was placed. Subsequently, VitreoDens was injected below the IOL or the crystalline lens remnant. BSS was stained with fluorescein to better visualize the VitreoDens-BSS-interface. To test the functionality as an intraoperative heavy liquid, left eyes of landrace and Göttingen Mini Pigs undergoing different in-vivo studies on the contralateral eye were used directly prior euthanasia. Ethical approval was gathered prior to the commencement of the study at the local review board. (Ethical approval number:) Eyes were vitrectomized and an iatrogenic retinal tear was induced using a 23G-cutter. Subsequently, BSS was instilled subretinally, the vitreous cavity was then filled with VitreoDens up to the posterior edge of the tear.

### Assessment of surgical time and waste reduction:

As no time-intensive PFDL-air-silicone oil exchange is necessary, a reduction in surgical time is to be expected. To test this hypothesis, fluid exchanges were simulated in-vitro. First, a composition comprising 80% w/w perfluorobutylpentane (F4H5), and 20% w/w polydimethylsiloxane (PDMS) (VitreoDens) or PFDL was injected. Subsequently, in case of VitreoDens, the remaining BSS was passively evacuated using a backflush. In case of PFD, a PFD-air exchange was performed and then 5000 mPas silicone oil was injected into the simulated vitreous cavity. The needed time in minutes was evaluated in triplicates. To quantify this, three surgeons performed the implantation of either perfluorcarbon liquid or VitreoDens and then either 1. Completely filled the vitreous cavity with VitreoDens or 2. Removed perfluorcarbon liquid against air including a 3-fold BSS wash, a maneuver to make sure to remove the PFCL without any remnants. Then perform a silicone oil fill including the setup of the high-pressure viscous fluid injection system. To visualize the reduction in waste, images of both approaches were taken prior to unpacking.

### In-vivo - Animals, ethical approval and experimental timeline:

A total of 8 male landrace and 3 male minipigs were used in the present study. Landrace pigs were sourced from a local farmer with an initial weight of 35 to 45 kg. Göttingen Mini Pigs were sourced from Ellegaard Gottingen Minipigs (Dalmose, Denmark). The experiment adhered to the ARVO Statement for the Use of Animals in Ophthalmic and Vision Research and was approved from the local ethics committee (ethical approval number: 35-9185.81/G-11/24). Pigs were housed in a specialized facility overseen by veterinarians, with water ad libitum and restricted food access. For all procedures, animals were sedated with an intramuscular injection of Sedanol (WDT, Garbsen, Germany) (6mg/kg) and then anaesthetized with ketamine (CP Pharma, Burgdorf, Germany) (11mg/kg) and midazolam (Hameln Pharma, Hameln, Germany) (2mg/kg). 3 landrace and the Göttingen Mini Pig were used for the long-term biocompatibility study. In 3 landrace pigs, additionally, an iatrogenic rhegmatogenous retinal detachment was created during surgery to prove the function as an endotamponade. 2 landrace pig and 2 Göttingen Mini Pigs were used for drug release studies utilizing VitreoDens loaded various active substances.

### In-vivo - Surgical procedure:

All procedures were performed on the right eye with sterile techniques under a surgical microscope (Leica, Wetzlar, Germany). After topical anaesthesia and pupil dilation, a standard three-port, 23-gauge vitrectomy system was used (Geuder AG, Heidelberg, Germany). The vitreous body was intraoperatively visualized with triamcinolone acetonide. After removal of the vitreous with a vitrectomy cutter, air-fluid exchange was performed in the vitreous cavity. Following air-fluid exchange, the vitreous cavity was filled with VitreoDens. This approach was kept to better compare results directly to established endotampoandes and to not impair the biocompatibility examination due to errors occuring due to the new VitreoDens implantation technique.

### In-vivo - Biocompatibility testing:

Biocompatibility testings of both eyes were conducted 2-, 4-, and 6-weeks postoperatively. Göttingen Mini Pigs were additionally examined at 10 and 12 weeks after surgery. On the surgical day, biocompatibility examinations were not performed to not impair visualization during surgery. First, intraocular pressure measurements were recorded using the iCare Tonovet Plus (Finland Oy, Vantaa, Finland). After inducing mydriasis (cyclopentolate (0,5%), epinephrine (5%) and tropicamide (1%)), fundus photographs of the posterior pole including the optical nerve head were acquired using the ClearView2 veterinary fundus camera (Optibrand, Fort Collins, USA). Subsequently, optical coherence tomography of the central retina was performed using the Spectralis Flex OCT (Heidelberg Engineering, Heidelberg, Germany). A 20° dense scan with a 30° infrared reflectance image was performed. To examine the exact same location at every subsequent biocompatibility visit, the follow-up function was used. Lastly, electroretinography was performed using the RETevet^{™} system (LKC Technologies, Gaithersburg, USA) and the Dog, Cat, Nonhuman Primate ISCEV 6 Step Light First test protocol. This test includes both, light- and dark-adapted tests (15 min dark adaptation), to better assess cone and rod function. The reference electrode was placed 2.5 cm lateral to the outer canthus. The active electrode was placed directly on the cornea. The ground electrode was placed centrally on the forehead.

### Histopathological Preparation:

All eyes that underwent the implantation of the composition comprising 80% *w*/*w* perfluorobutylpentane (F4H5), and 20% *w*/*w* polydimethylsiloxane (PDMS) (VitreoDens) were enucleated post-mortem and processed for either H&E or immunostaining with Iba1 (microglia), GFAP (possible proliferation of Muller glia-cells) or CD45 (immune cell infiltration). Details on the staining procedures are presented in the Supplementary Materials section.

### Chosen drugs for the evaluation of VitreoDens as an intraocular controlled release system:

**Table 1 showcases the different drugs evaluated. The Electronic Supplementary outlines the origin of the materials.**

| **Classes** | **Drugs evaluated** |
|---|---|
| Antibiotics | Ceftazidime, Vancomycin |
| Antifungals | Voriconazole |
| Steroids | Dexamethason |
| Immunomodulation | Cyclosporin A, Tacrolimus |
| Anti-Glaucomatous | Latanoprost, Bimatoprost and Timolol-maleate |
| Antiproliferative | 5-FU, Methotrexate, Daunorubicin, Idarubicin |

Drugs were dissoluted in BSS in clinically revelant concentrations and placed in direct contact with VitreoDens in a ratio of 1:9. 50 µl of the hydrophilic phase were removed from the experiment after 1, 6 and 24 hours and drug concentration was determined using HPLC.

### Quantification of drug levels:

For all the tested small molecule drugs HPLC or HPLC -MS (Agilent 1100 Series) with a reversed-phase C18 column (Chromolith^{®} Performance RP-18e, Merck KGaA Darmstadt, Germany) was performed. Calibration curves and exemplary HPLC runs for each active substance are presented in the Supplementary Materials.

### In-vitro drug release model:

For drugs soluble in VitreoDens, the drug was dissolved in VitreoDens and a release-profile was created. To simulate the composition of the posterior segment, a previously used simple posterior segment model as depicted in Fig. 8A was used. In short, 4.5 ml of VitreoDens were incubated with 0.5 ml of BSS (indicating a 90% fill of the vitreous cavity). The drug was dissolved in VitreoDens prior to the initiation of the release model. Every 24 hours the hydrophilic phase is replaced to simulate aqueous flow. After 6 and 24 hours, 50 µl of sample were analyzed using HPLC or HPLC-MS.

### Statistical analysis:

The data were processed and analyzed with Stata BE (StataCorp, College Station, Texas, USA) or Prism 10 (GraphPad Inc., California, USA. Continuous data were presented as the means ± SEM of at least three independent experiments. The distributions of continuous variables were assessed by inspecting histograms and using Kolmogorov-Smirnov tests to assess normality. T-tests or Mann-Whitney-tests were applied as appropriate. Values less than 0.05 were considered statistically significant.

### Results

**Table 2 summarizes the results of the measurements of refractive indexes of Examples 1-7. The mean value for the refractive index is given.**

| **Sample** | **Refractive Index** |
|---|---|
| Mixture A | 1.3256 |
| Mixture B | 1.3261 |
| Mixture C | 1.3404 |
| Mixture D | 1.3455 |
| Mixture E | 1.3620 |
| Mixture F | 1.3785 |
| Mixture G | 1.3784 |

In summary, the Examples demonstrate that it is possible to provide compositions comprising a mixture of at least one oil and one or more semifluorinated alkanes, wherein the refractive index of the composition is from about 1.310 to about 1.365 at 35°C (Mixture A, Mixture B, Mixture C, Mixture D and Mixture E), thus being closer to the refractory index of the vitreous body than e.g. commonly used tamponade silicone oils, thereby overcoming or at least reducing the issues related to visual acuity of patients as described above during application of a tamponade.

### Example 8 The physico-chemical properties of compositions according to the present invention are precisely tunable:

The relation between the percentage of F4H5, refractive index and density is linear (Fig. 1 A-C) allowing the precise tuning of VitreoDens to exhibit the exact refractive index of the vitreous body at 35 °C (Fig 1 D). Specifically, Fig 1A shows the relation between the density of a composition and the refractive index of the composition for compositions having different F4H5:PDMS ratios. Fig. 1B shows the relation between the percentage of F4H5 of a composition and the refractive index of the composition for compositions having different F4H5: PDMS ratios. Fig. 1C shows the relation between the percentage of F4H5 of a composition and the density of a composition for compositions having different F4H5: PDMS ratios. Fig. 1D shows the refractive index of compositions according to embodiments of the present invention in comparison to the refractive index of currently used endotamponades and heavy liquids according to the state of the art. In particular, "PFD" relates to Perfuorodecalin, "VB" relates to "vitreous body", "VD1000" relates to a composition comprising 80% w/w perfluorobutylpentane (F4H5), and 20% w/w polydimethylsiloxane (PDMS), wherein the PDMS has an average viscosity of about 1000mPas at 25°C. "VD5000" relates to a composition comprising 80% w/w perfluorobutylpentane (F4H5), and 20% w/w polydimethylsiloxane (PDMS), wherein the PDMS has an average viscosity of about 5000mPas at 25°C. "VD HMWC" relates to a composition comprising 80% w/w perfluorobutylpentane (F4H5), and 20% w/w polydimethylsiloxane (PDMS), wherein the PDMS has an average viscosity of about 2.5 million mPas at 25°C. "D68" relates to Densiron^{®} 68 - (Fluoron GmbH), and "Sil" relates to Silicone oil. Fig. 1E shows the density of a composition comprising 80% w/w perfluorobutylpentane, and 20% w/w polydimethylsiloxane (VitreoDens) compared to currently available long-term endotamponades and heavy liquids. The abbreviations used for the compounds tested in Fig 1E are identical to the abbreviations used in Fig 1D (see above). Fig. 1F demonstrates that the viscosity of VitreoDens can be freely tuned between 7 and 2481 mPas by varying the molecular weight of the PDMS-component. Specifically, the triangle relates to a composition comprising 80% w/w perfluorobutylpentane (F4H5), and 20% w/w polydimethylsiloxane (PDMS), wherein the PDMS has an average viscosity of about 1000mPas at 25°C (Vitrodens 1000). the square relates to a composition comprising 80% *w*/*w* perfluorobutylpentane (F4H5), and 20% *w*/*w* polydimethylsiloxane (PDMS), wherein the PDMS has an average viscosity of about 5000mPas at 25°C (Vitrodens 5000), and the rhombus relates to a composition comprising 80% *w*/*w* perfluorobutylpentane (F4H5), and 20% *w*/*w* polydimethylsiloxane (PDMS), wherein the PDMS has an average viscosity of about 2.5 million mPas at 25°C (Vitrodens HMWC). Fig. 1G shows that VitreoDens holds an extremely low level of turbidity. The abbreviations used for the compounds tested in Fig 1H are identical to the abbreviations used in Fig 1D (see above). Fig. 1H shows that VitreoDens holds an extremely low level of forward light scattering compared to other biomaterials used in ophthalmology. "VD1000" relates to a composition comprising 80% *w*/*w* perfluorobutylpentane (F4H5), and 20% *w*/*w* polydimethylsiloxane (PDMS), wherein the PDMS has an average viscosity of about 1000mPas at 25°C. "VD5000" relates to a composition comprising 80% *w*/*w* perfluorobutylpentane (F4H5), and 20% *w*/*w* polydimethylsiloxane (PDMS), wherein the PDMS has an average viscosity of about 5000mPas at 25°C. "VD HMWC" relates to a composition comprising 80% *w*/*w* perfluorobutylpentane (F4H5), and 20% *w*/*w* polydimethylsiloxane (PDMS), wherein the PDMS has an average viscosity of about 2.5 million mPas at 25°C. "Hydrophobic IOL" relates to hydrophobic intraocular lenses, and "hydrophilic IOL" relates to hydrophilic intraocular lenses. Fig. 1I demonstrates that the transmission spectra of VitreoDens showes excellent results allowing every relevant wavelength to either image or laser-treat the retina to pass freely.

### Example 9 Compositions according to the present invention are highly emulsification-resistant and non-cytotoxic in ARPE-19 and L-929 cell cultures:

A composition comprising 80% *w*/*w* perfluorobutylpentane (F4H5), and 20% *w*/*w* polydimethylsiloxane (PDMS) (VitreoDens) was evaluated on its tendency to emulsify based on its electrochemical emulsion stability. Fig. 2A graphically illustrates the principle correlation between a composition's tendency to emulsify and its electrochemical emulsion stability using zeta-potential. The higher the electrochemical emulsion stability, the likelier an emulsion droplet will stay and not return to the main phase (A - I). If emulsion droplets are less stable, they can more easily unify with the main oil bubble (A - II). As shown in Fig. 2B, VitreoDens showed a favorable electrochemical emulsion stability (p<0.0001 compared to Siluron 5000, t-test, n= 3(9)). In particular, in Fig. 2B, "VD1000" relates to a composition comprising 80% *w*/*w* perfluorobutylpentane (F4H5), and 20% *w*/*w* polydimethylsiloxane (PDMS), wherein the PDMS has an average viscosity of about 1000mPas at 25°C. "VD5000" relates to a composition comprising 80% *w*/*w* perfluorobutylpentane (F4H5), and 20% *w*/*w* polydimethylsiloxane (PDMS), wherein the PDMS has an average viscosity of about 5000mPas at 25°C. "VD HMWC" relates to a composition comprising 80% *w*/*w* perfluorobutylpentane (F4H5), and 20% *w*/*w* polydimethylsiloxane (PDMS), wherein the PDMS has an average viscosity of about 2.5 million mPas at 25°C. "D68" relates to Densiron^{®} 68 - (Fluoron GmbH), and "S5000" relates to Siluron 5000 (Fluoron GmbH). Further, using the Caramoy-Assay as schematically explained in Fig. 2C, VitreoDens proved to ultra emulsification-resistant compared to current clinically used endotamponades (p<0.002 compared to Siluron 5000, t-test, n=3, see Fig. 2D). Specifically, in Fig 2D, "VD5000" relates to a composition comprising 80% *w*/*w* perfluorobutylpentane (F4H5), and 20% *w*/*w* polydimethylsiloxane (PDMS), wherein the PDMS has an average viscosity of about 5000mPas at 25°C, "D68" relates to Densiron^{®} 68 - (Fluoron GmbH), and "S5000" relates to Siluron 5000 (Fluoron GmbH). "Further, Fig 1E demonstrates that VitreoDens showcasts a high interfacial tension to balanced salt solution in line with current endotamponades. The abbreviations used for the compounds tested in Fig. 2E are identical to the abbreviations used in Fig. 2B (see above). VitreoDens was tested in cell cultures using both the direct and transwell approach as schematically depicted in Fig 2F with 3 different molecular weights for the PDMS component (1000 mPas, 5000 mPas and 2.5 mio mPas). VitreoDens is highly biocompatible and non-cytotoxic to human retinal pigment epithelium (Direct contact - O, Transwell - M, both N=3 and with subsequent n=3) and L929-fibroblast cells according to **DIN EN** ISO 10993-5: 2009. Specifically, Fig. 2G shows the cell viability of compositions according to an embodiment of the present invention comprising 80% *w*/*w* perfluorobutylpentane (F4H5), and 20% *w*/*w* polydimethylsiloxane (PDMS), wherein "D45 1000" relates to a composition comprising 80% *w*/*w* perfluorobutylpentane (F4H5), and 20% *w*/*w* polydimethylsiloxane (PDMS), wherein the PDMS has an average viscosity of about 1000mPas at 25°C. "D45 5000" relates to a composition comprising 80% *w*/*w* perfluorobutylpentane (F4H5), and 20% *w*/*w* polydimethylsiloxane (PDMS), wherein the PDMS has an average viscosity of about 5000mPas at 25°C. "D45 HMWC" relates to a composition comprising 80% *w*/*w* perfluorobutylpentane (F4H5), and 20% *w*/*w* polydimethylsiloxane (PDMS), wherein the PDMS has an average viscosity of about 2.5 million mPas at 25°C. Fig. 2 H shows the cell viability of compositions according to an embodiment of the present invention comprising 80% *w*/*w* perfluorobutylpentane (F4H5), and 20% *w*/*w* polydimethylsiloxane (PDMS), wherein "VD1000" relates to a composition comprising 80% *w*/*w* perfluorobutylpentane (F4H5), and 20% *w*/*w* polydimethylsiloxane (PDMS), wherein the PDMS has an average viscosity of about 1000mPas at 25°C. "VD5000" relates to a composition comprising 80% *w*/*w* perfluorobutylpentane (F4H5), and 20% *w*/*w* polydimethylsiloxane (PDMS), wherein the PDMS has an average viscosity of about 5000mPas at 25°C. "VD HMWC" relates to a composition comprising 80% *w*/*w* perfluorobutylpentane (F4H5), and 20% *w*/*w* polydimethylsiloxane (PDMS), wherein the PDMS has an average viscosity of about 2.5 million mPas at 25°C. Thus, the abbreviations "D45" in Fig 2G and "VD" in Fig. 2H are used equivalently to relate to a composition comprising 80% *w*/*w* perfluorobutylpentane (F4H5), and 20% *w*/*w* polydimethylsiloxane (PDMS). Fig. 2I shows the cell viability of VD1000, VD5000 and VD HMWC, using different percentages.

### Example 10 VitreoDens functions as both an intraoperative heavy liquid and postoperative endotamponade:

### VitreoDens can be easily injected and removed from the vitreous cavity:

The primary way of application of heavy liquids in vitreoretinal surgery is by injecting the liquid in the BSS-filled eye by hand to reattached the retina or lift intraocular lenses or crystalline lens fragments off the retina (Fig. 3 A-C). The injection force needed to inject VitreoDens 1000 and VitreoDens 5000 was comparable to currently used heavy liquid restricted to the intraoperative use (Fig. 3. E and G). VitreoDens forms a coherent liquid bubble inside the vitreous cavity that can be easily aspirated using a standard 23G-27G cutter even with a very low active aspiration such as 100 mmHg within under a minute. (Fig. 3. F and H). Specifically, in Fig 3F, "F4H5" relates pure F4H5^{®} (Fluoron GmbH), "F-Octan" relates to F-Octan (Fluoron GmbH), "F-Decalin" relates to F-Decalin (Fluoron GmbH), "VD1000" relates to a composition comprising 80% *w*/*w* perfluorobutylpentane (F4H5), and 20% *w*/*w* polydimethylsiloxane (PDMS), wherein the PDMS has an average viscosity of about 1000mPas at 25°C. "VD5000" relates to a composition comprising 80% *w*/*w* perfluorobutylpentane (F4H5), and 20% *w*/*w* polydimethylsiloxane (PDMS), wherein the PDMS has an average viscosity of about 5000mPas at 25°C. "VD HMWC" relates to a composition comprising 80% *w*/*w* perfluorobutylpentane (F4H5), and 20% *w*/*w* polydimethylsiloxane (PDMS), wherein the PDMS has an average viscosity of about 2.5 million mPas at 25°C. "S1000" relates to Siluron^{®} 1000 - Fluoron GmbH. Interestingly, due to the optimized refractive index the interface itself is barely visible, however, a shadow cast on the retina allows visualization.

### VitreoDens acts as an intraoperative heavy liquid in-vitro and in-vivo:

The two core functions of intraoperative heavy liquids utilized during vitreoretinal surgery is the ability to lift remnants of the crystalline lens or intraocular lens implants and the reattachment of the neuroretina on the retinal pigment epithelium (RPE) (Fig. 3. A and B). Due to its density, VitreoDens fulfills both of the criteria in vitro and in vivo: VitreoDens is able to lift remnants of the human and porcine crystalline lens as well as commonly used intraocular lenses. When pressure is applied, the material does not sink and is safely kept at the interface between VitreoDens and the hydrophilic phase. (Fig. 3. C) demonstrates this ability in an in-vitro setup. Further, VitreoDens is capable to act as a heavy liquid to reattach the retina as depicted in Fig. 3. I *in vivo.* Another characteristic of intraoperative heavy liquids is their unrestrained transmission for wavelengths used for intraoperative laser treatments of the retina. This ability is matched by VitreoDens (Fig. 3. J).

### VitreoDens acts as a postoperative endotamponade in pigs:

To fully showcase the dual use function of VitreoDens as a heavy liquid and as a postoperative endotamponade, 3 male landrace pigs were vitrectomized and an iatrogenic retinal tear was placed and subsequently irrigated with balanced salt solution to create an iatrogenic, rhegmatogenous retinal detachment. VitreoDens was used to reattach the retina and the vitreous cavity was filled with VitreoDens. 2, 4 and 6 weeks after surgery pigs were reevaluated. In all 3 animals, the retina was completely attached proving the function as an endotamponade. Fig. 3D showcases the effective tamponading effect of VitreoDens and the high surface tension required to bridge large retinal holes.

The main function of an intraoperative heavy liquid is to evacuate subretinal fluid (Fig. 3A) and to lift foreign bodies away from the posterior pole to protect the retina (Fig 3B). VitreoDens can lift IOLs (Fig 3C, left) and crystalline lenses (Fig 3C, right). BSS was stained with fluorescein to better visualize the interface. Fig 3D demonstrates that VitreoDens effectively tamponades retinal breaks as seen on the optical coherence tomography B-scan of a pig 2 weeks after inducing an iatrogenic retinal detachment followed by a VitreoDens-tamponade. Fig 3E shows that the Injection Force needed to inject VitreoDens is comparable to clinically used heavy liquids and easily achievable by hand. Fig 3F demonstrates that VitreoDens can be removed from the vitreous cavity within minutes using active aspiration through a standard 23G-cutter. Fig 3G shows that VitreoDens can easily be injected by hand and forms a coherent bubble inside the vitreous cavity only visible by a shadow cast onto the retina. Fig 3H demonstrates that VitreoDens can be removed with a standard 23G-cutter. Fig 3I shows that VitreoDens effectively reattaches the neuroretina in vivo in pigs. Fig 3J demonstrates that Endolaser procedures can be performed during a VitreoDens fill.

### Example 11 VitreoDens exhibits excellent long-term biocompatibility over 3 months in-vivo Morphology:

8 landrace pigs and one Göttingen Mini Pig were successfully vitrectomized, implanted with VitreoDens and observed for 6 or 12 weeks respectively with a biweekly biocompatibility assessment (Fig. 4 A). Fig 4B shows exemplary fundus photographs of the right eye of a Göttingen Mini Pig implanted with VitreoDens. Retinal structure and vessels do not show any signs of inflammation or atrophy. Retinal view remained unobstructed as no cataract developed. All animals showed no signs of intraocular inflammation. Specifically, no signs of vessel sheathing during fundoscopy (Fig. 4B). Fig 4C shows exemplary OCT b-scans of the same Göttingen Mini Pig at 2, 4, 6 and 12 weeks after vitrectomy with the implantation of VitreoDens. No retinal edema was detected using optical coherence tomography (Fig. 4 C), and no increased iris vasculature were observed during the examination. Two weeks postoperatively, no anterior chamber or vitreous inflammation were detectable.

Post-mortem HE-stainings of the retina showed excellent retinal architecture (Fig. 4 D). Immunostainings of CD45 (to detect infiltrating immune cells), IBA1 (to detect microglial activation) and GFAP (to detect Müller glia activation) showed no increased retinal inflammation (Fig. 4 E).

Further, no emulsification-related issues occurred during the whole study period. In detail, no emulsified droplets were detected in the anterior chamber, no signs of corneal edema or corneal decompensation detected by OCT and images of the anterior chamber angle showed no relevant thickening of trabecules and no inflammation (Fig 4 F and G). Retinal thickness was preserved throughout the whole study period (Fig. 4 H).

### Example 12 VitreoDens is highly biocompatible for 12 weeks in an in vivo pig model - Function:

Current endotamponades cause a great refractive shift due to their non-optimized refractive index (Fig 5A). VitreoDens was purposefully designed to exhibit the refractive index of the vitreous body at 35°C (Fig 5A), not leading to any refractive shift compared to the control eye (p=0.65) (Fig 5B). Specifically, in Fig 5B, "R" relates to the treated right eye and "L" relates to the non-treated left eye of the animal, wherein only one of both eyes was treated. The Spherical Equivalent (SpH Equ [D]) in treated and non-treated eyes of animals has been measured 2 weeks post-op and 6/12 weeks post-op, respectively, and is indicated in Fig 5B. Fig 5C shows that intraocular pressure remained controlled in all animals over the study duration. Black circles relate to measurements of the non-treated left eye, and white circles relate to measurements of the treated right eye, respectively. Fig 5D and Fig 5E, respectively, demonstrate that A and B-wave latencies and ERG amplitudes did not statistically differ between 2 and 6-12 weeks after VitreoDens implantation. Black circles relate to measurements of the non-treated left eye, and white circles relate to measurements of the treated right eye, respectively. Fig 5F and Fig 5G, respectively, show Light- and Dark-adapted exemplary eletroretinographical responses compared between 2 and 12 weeks after the implantation of VitreoDens.

The intraocular pressure was regulated in all animals throughout the study period, see Fig. 5C. Lastly, retinal function was fully preserved in all animals at the end of the study period quantified using electroretinography with no significant changes in a or b-wave amplitude or latency in light and dark-adapted tests (Fig. 5 D-E). Apart from slight a posterior capsule opacification most likely due to surgical trauma, no cataract development was visible and posterior segment visualization was unobstructed at all times.

### VitreoDens does not cause a refractive shift in-vivo

Using a hand-held refractometer and the focus function of the Spectralis OCT, a postoperative refractive shift compared to the control eye was determined. Compared to the known refractive shift of currently clinically used endotamponades of between 6-10 diopters (Fig. 5 A), VitreoDens did not cause a significant refractive shift compared to preoperative values and compared to the partner eye (Fig. 5A-B).

### Example 13 VitreoDens shortens surgical time significantly, reduces surgical waste and is environmentally friendly:

### VitreoDens majorly reduces surgical time:

A major downside of the use of PFDL as a heavy liquid during vitreoretinal surgery is its compromised biocompatibility limiting its use to the intraoperative time frame. As long-term endotamponades like silicone oils are soluble in perfluordecalin, caution has to be taken when exchanging the two substances. Thus, common practice is not a direct exchange but the replacement of perfluordecalin with an air tamponade follow by one to three wash cycles with balanced salt solution as even small remnants of perfluordecalin can induce central retinal atrophy. Subsequently, the final tamponade, e.g. silicone oil, is injected using a viscous fluid injection system (Fig. 6 A). As VitreoDens can be used during both, the intra- and postoperative time frame, no exchange of fluids is necessary effectively shortening surgical times. Three experienced retinal surgeons performed either PDFL-silicone oil exchange as described above or a VitreoDens filling in an in-vitro setting (to better allow comparison to not be influenced by visualization). Fully filling the simulated vitreous cavity after completing the surgery took roughly 60 seconds, compared to roughly 7 minutes for the PDFL-silicone oil exchange process (Fig. 6 C). Given a surgical time of around 35 minutes for retinal detachment surgery treated with a silicone oil endotamponade, this corresponds to a 14.3% surgical time reduction.

### VitreoDens majorly reduces surgical waste:

As described previously, for PFDL-silicone oil exchange requires an intermittent air tamponade and the subsequent injection of viscous silicone oil requiring a specialized 6-bar pressure viscous fluid injection system. Fig. 6 B showcases the amount of waste generated by single use instruments utilized during a PFDL-silicone oil exchange and a VitreoDens filling, respectively. Next to waste reduction, this may translate to significant cost savings.

### Example 14 VitreoDens can uptake and release clinically a relevant amount of various important drugs applied intravitreally:

VitreoDens can take up clinically relevant dosages of various drugs:
F4H5 has been recently discovered as a drug carrier, specifically for the topical administration of immunomodulatory drugs to the surface of the eye. Thus, the solubility of a variety of drugs was tested in VitreoDens in an established posterior segment model (Fig. 7 A and B) A >90% fill is mostly thought of as a sufficient fill. Thus, this ratio, given a vitreous cavity volume of 5 ml, was recreated in negatively charged glass vials. First, the drug is injected into the hydrophilic phase at a certain concentration, aliquots of the hydrophilic phase are recovered after certain time points to assess the distribution of the drug in the hydrophilic phase and in VitreoDens (Fig. 7A). For drugs, that showed solubility, the experiment is reversed to evaluate the release potential of VitreoDens (Fig 7B). Of the candidates, the immunomodulatory drugs Cyclosporin and Tacrolimus, the antiproliferative substances 5-Fluoruracil and Idarubicin as well as the antimycotic voriconazole showed excellent solubility in VitreoDens in-vitro (Fig. 7 C).

VitreoDens acts as an intraocular sustained drug release system in-vitro:
For a variety of drugs, the release properties of VitreoDens were tested in-vitro. Again, the established posterior segment model was utilized. Similar to the abovementioned uptake study, results varied mostly based on the lipophilicity of the drugs. The release curves of voriconazole, tacrolimus and cyclosporin are shown in Fig. 7D-F. Specifically, the release curves for a 21-day experiments are shown for voriconazole (Fig 7D), cyclosporin (Fig 7E) and tacrolimus (Fig 7F).

### Example 15 Parameters of different VitreoDens batches:

**Table 3:**

| *Test parameter Sterility* | *Acceptance Criteria* sterile | ***Unit*** - | Batch 1 **sterile (no macroscopic growth visible)** | Batch 2 **sterile** | Batch 3 **n.a.** |
|---|---|---|---|---|---|
| *Endotoxine* | <0,2 EU/ml | **EU/ml** | **<0,04** | **<0,03** | **n.a.** |
| L929 *Cytotoxicity* | not cytotoxic | - | **not cytotoxic** | **not cytotoxic** | **n.a.** |
| ARP19 *Cytotoxicity* | not cytotoxic | - | **not cytotoxic** | **not cytotoxic** | **n.a.** |
| *surface tension* [35°C] | tbd. | **mN/m** | **15,7** | **15,8459 ± 0,0157** | **15,6234 ± 0,0799** |
| *density* [35°C] | tbd. | **g/cm3** | **1,19** | **1,186** | **1,188** |
| *transmission* | tbd. - Ideal: nearly Baseline as PFCLs | - | **Minimal Peak (ca. 5%) at ca. 910 nm** | **Minimal Peak (ca. 5%) at ca. 910 nm** | **Minimal Peak (ca. 5%) at ca. 910 nm** |
| *refractive index* [35°C] | Water [35°C]: 1,331 tbd. | - | **1,3355** | **1,3360** | **1,3345** |
| *viscosity* [35°C] | tbd. | | **16,7 cSt (measured) 19,9 mPas (calculated)** | **19,456 cSt (measured) 23,07 mPas (calculated)** | **n.a.** |
| *turbidity room temperature* | tbd. | **NTU** | **0,92** | **0,88** | **0,88** |
| *interfacial tension* | interim specification [35°C]: >40 mN/m | **mN/m** | **42,6** | **41,4880 ± 0,0836** | **42,7722 ± 0,0790** |

It will be readily apparent to a person skilled in the art that varying substitutions and modifications may be made to the invention disclosed herein without departing from the scope and spirit of the invention.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

The inventions illustratively described herein may suitably be practiced in the absence of any element or elements, limitation or limitations, not specifically disclosed herein. Thus, for example, the terms "comprising", "including", "containing", etc. shall be read expansively and without limitation. The term "VitreoDens" as used herein is used equivalently to relate to a composition comprising 80% *w*/*w* perfluorobutylpentane (F4H5), and 20% *w*/*w* polydimethylsiloxane (PDMS). Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention. The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein. **In** addition, where features or aspects of the invention are described in terms of Markush groups, those skilled in the art will recognize that the invention is also thereby described in terms of any individual member or subgroup of members of the Markush group. Further embodiments of the invention will become apparent from the following claims.

## Claims

1. A composition comprising a mixture of at least one oil and one or more semifluorinated alkanes, the refractive index at 589 nm at 35°C of the composition being from about 1.310 to about 1.365.

2. The composition according to claim 1, wherein the refractive index is from about 1.315 to about 1.360, such as from about 1.320 to about 1.350, preferably wherein the refractive index is from about 1.325 to about 1.345, such as from about 1.330 to about 1.340, most preferably wherein the refractive index is about 1.331.

3. The composition according to claim 1 or 2, wherein the one or more semifluorinated alkane comprises perfluorobutylpentane (F4H5), perfluorobutylhexane (F4H6), perfluorobutyloctane (F4H8), perfluorohexylhexane (F6H6), perfluorohexyloctane (F6H8) or a mixture thereof, preferably the semifluorinated alkane being perfluorobutylpentane (F4H5).

4. The composition according to any one of claims 1- 3, wherein the at least one oil is selected from one or more of the groups consisting of a silicone oil, a fluorosilicone oil, a silicone-fluorosilicone copolymer oil, a silicone-phenylsilicone copolymer oil, a silicone oil derivative and a mixture thereof, preferably wherein the at least one oil is a silicone oil.

5. The composition according to any one of claims 1-4, wherein the one or more semifluorinated alkanes are present in the composition in a quantity in a range of about 56% *w*/*w* - about 99% *w*/*w.,* or of about 70% w/w- about 90 % *w*/*w,* or of about 75.1 % *w*/*w* - about 90% *w*/*w,* preferably of about 77% *w*/*w* - about 85% *w*/*w,* based on the total weight of the composition.

6. The composition according to any one of claims 1-5, wherein the at least one oil is present in the composition in a quantity in the range of about 1% w/w - about 44% w/w, or of about 10% w/w - about 30% w/w, or of about 10% w/w - about 24.9 % w/w, preferably about 15% w/w - about 23% w/w, based on the total weight of the composition.

7. The composition according to any one of claims 1 - 6, wherein the at least one oil comprises an oil having a viscosity of about 9,999 mPas or less, such as e.g. of about 7,000 mPas or less; and/or wherein the at least one oil comprises an oil having a viscosity in the range of about 10,000-25,000,000 mPas.

8. The composition according to claim 7, wherein the at least one oil comprises an oil having a viscosity of about 1,000 mPas, and/or of about 2,000 mPas, and/or of about 5,000 mPas.

9. The composition according to claim 7 or 8, wherein the at least one oil comprises at least a first oil and a second oil,
wherein the first oil has a viscosity of about 9,999 mPas or less, such as of about 7,000 mPas or less; and further wherein the second oil has a viscosity in the range of about 10,000-25,000,000 mPas,
or wherein the first oil and the second oil have a viscosity about 9,999 mPas or less, such as about 7,000 mPas or less,
or wherein the first oil and the second oil have a viscosity in the range of about 10,000-25,000,000 mPas.

10. The composition according to claim 9, wherein the first oil is present in the composition in a quantity in the range of about 1% w/w - about 43 % w/w, and wherein the second oil is present in the composition in a quantity in a range of about 1% w/w - about 43 % w/w, based on the total weight of the composition.

11. The composition according to any one of the preceeding claims, wherein the composition further comprises additives selected from one or more of emulsification inhibitors, steroids, non-steroidal anti-inflammatory agents, antibiotics or a mixture thereof, preferably wherein the additive is present in the composition in a quantity in a range of about 0.0001 to about 10 % w/w, optionally from about 0.1 to about 5 % w/w, based on the weight of the total composition.

12. The composition according to any one of the preceeding claims, further wherein the composition comprises about 80% w/w perfluorobutylpentane (F4H5), and about 20% w/w polydimethylsiloxane (PDMS), based on the total weight of the composition.

13. The composition according to any one of the preceeding claims for use in the treatment of a disease, such as the treatment of a detached retina.

14. A kit of parts comprising the composition of any one of claims 1 to 12 in a vial or syringe, one or more of cannulas and/or tubing, and instructions for use.

15. A method of producing a composition for use in the treatment of a disease, optionally comprising treatment of a detached retina, comprising mixing at least one oils and one or more semifluorinated alkanes to produce a composition of any one of claims 1 to 12.
